# EUROPEAN PATENT SPECIFICATION

(11) **EP 2 561 083 B1**
(45) Date of publication and mention of the grant of the patent: **11.01.2017**
(21) Application number: 11755159.8
(22) Date of filing: 05.08.2011
(51) Int. Cl.: C12P 19/14, C12P 19/02

(54) **Use of Humicola grisea glucoamylase in an SSF process at neutral pH**
Verwendung von Humicola grisea Glucoamylase in einem SSF-Verfahren bei neutralem pH
Utilisation d'une Humicola grisea glucoamylase dans un procédé SSF de pH neutre

(30) Priority: 06.08.2010 US 371639 P
(43) Date of publication of application: 27.02.2013
(73) Proprietor: Danisco US Inc., Palo Alto, CA 94304-1013 (US)
(72) Inventor: BERGSMA, Martien, H., CA 94304-1013 (US); CHOTANI, Gopal, K., CA 94304-1013 (US); CUEVAS, William, A., CA 94304-1013 (US); DUAN, Gang, CA 94304-1013 (US); LEE, Sung, Ho, CA 94304-1013 (US); QIAN, Ying, CA 94304-1013 (US); SHARMA, Vivek, CA 94304-1013 (US); SHETTY, Jayarama, K., CA 94304-1013 (US); STROHM, Bruce, A., CA 94304-1013 (US); TEUNISSEN, Pauline Johanna, Maria, CA 94304-1013 (US); XU, Hongxian, CA 94304-1013 (US)
(74) Representative: Kiddle, Simon John
(86) International application number: PCT/US2011/046849
(87) International publication number: WO 2012/019159

(56) References cited:
- EP-A1- 0 186 066
- EP-A2- 0 171 218
- WO-A1-2009/052101
- WO-A2-2006/060062
- US-A1- 2005 208 623
- KIM ET AL: "Properties of a novel thermostable glucoamylase from the hyperthermophilic archaeon Sulfolobus solfataricus in relation to starch processing", APPLIED AND ENVIRONMENTAL MICROBIOLOGY, vol. 70, 2004, pages 3933-3940, XP002664546,
- HUANG ET AL: "Simultaneous saccharification and fermentation of potato starch wastewater to lactic acid by Rhizopus oryzae and Rhizopus arrhizus", BIOCHEMICAL ENGINEERING JOURNAL, vol. 23, 2005, pages 265-276, XP027849102,

## Description

### FIELD OF THE INVENTION

The invention refers to the use of a Humicola grisea glucoamylase at pH 6.0 to 7.5 in an SSF process.

### BACKGROUND

Industrial fermentations predominately use glucose as a feedstock for the production of a multitude of proteins, enzymes, alcohols, and other chemical end products. Typically, glucose is the product of starch processing, which is conventionally a two-step, enzymatic process that catalyzes the breakdown of starch, involving liquefaction and saccharification. During liquefaction, insoluble granular starch is slurried in water, gelatinized with heat, and hydrolyzed by a thermostable alpha-amylase. During saccharification, the soluble dextrins produced in liquefaction are further hydrolyzed by glucoamylases producing a high glucose syrup containing greater than 95 % glucose.

Glucoamylases are exo-acting carbohydrases, capable of hydrolyzing both the linear and branched glucosidic linkages of starch (*e.g*., amylose and amylopectin). Commercially, glucoamylases are typically used in the acidic pH ranges (pH less than 5.0) to produce fermentable sugars from the enzyme liquefied starch substrate. The fermentable sugars, *e.g*., low molecular weight sugars, such as glucose, may then be converted to fructose by other enzymes (*e.g*., glucose isomerases); crystallized; or used in fermentations to produce numerous end products (*e.g*., alcohols, monosodium glutamate, succinic acid, vitamins, amino acids, 1,3-propanediol, and lactic acid).

A system that combines (1) saccharification and (2) fermentation is known as simultaneous saccharification and fermentation (SSF). SSF replaces the classical double-step fermentation, i.e., production of fermentable sugars first and then conducting the fermentation process for producing the end product. In SSF, an inoculum can be added along with the starch hydrolyzing enzymes to concurrently saccharify a starch substrate and convert the saccharification products (*i.e*., fermentable sugars) to the desired end product. The inoculum is typically a microorganism capable of producing the end product. The benefits of SSF include, but are not limited to, the following:
**1)** promote sustained microbial growth by providing a continuous feeding of glucose;
**2)** improving the carbon conversion efficiency by reducing the osmotic stress;
**3)** boosting fermentation capacity by accommodating higher dry solids; and
**4)** improving end product yield and facilitating downstream processing due to reduced production of reversion reaction products and non-fermentable sugars.
SSF is particularly promising where a high concentration substrate is present in a low reactor volume. *See e.g*., John et al., Biotechnol. Adv. 27: 145-152 (2009).

There is still need to optimize SSF by choosing conditions, e.g., temperature, pH, enzymes, etc., that are most suitable for both saccharification and fermentation. For example, the pH of the yeast fermentation matches the saccharifying glucoamylase enzyme activity during the production of fuel alcohol using grain as a feedstock. The need is acute particularly for fermentations that are optimally performed above pH 6.0. Most commercial saccharification enzymes, *e.g*., *Aspergillus niger* glucoamylase (AnGA), only display significant saccharifying enzyme activity in the pH range of 4.2 to 5.5. The glucoamylases display significantly lowered activity at the fermentation pH above 6.0. Additional steps, such as pre-treatment or pre-saccharification for producing fermentation feed stocks, have been used when the optimal conditions for the fermentation and saccharification are not congruent. As disclosed in WO 2003/066816, for example, the slurry is subject to pasteurization at 65°C for 14 hours before the SSF is performed to produce 1,3-propanediol at 34°C. Similarly, the lactic acid-producing microorganism may be subject to forty to fifty serial transfers at an acidic pH before being applied in the SSF to produce lactic acid. *See* WO 2003/095659.

US 2005/0208623 (Baldwin et al) refers to fungal host cells for producing enzymes having glucoamylase activity, and discloses in figure 8 the stability of Humicola grisea glucoamylase at different pH values. EP 0 171 218 (Genencor) discloses fungal enzyme preparations, e.g. from Humicola grisea, for hydrolysis of granular starch to glucose.

### SUMMARY OF THE INVENTION

Accordingly, the present invention provides a method of processing starch in a simultaneous saccharification and fermentation (SSF) process comprising saccharifying a starch substrate to fermentable sugars at pH 6.0 to 7.5 in the presence of a glucoamylase, wherein the glucoamylase possesses at least 50% activity at pH 6.0 or above relative to its maximum activity, wherein the glucoamylase is *Humicola grisea* glucoamylase (HgGA) comprising SEQ ID NO: 3, or a variant thereof having at least 99% sequence identity to SEQ ID NO: 3, and fermenting the fermentable sugars to an end product. This property enables HgGA to be used in simultaneous saccharification and fermentation (SSF) to produce end products from a starch substrate.

In one aspect, the starch substrate is from corn, wheat, rye, barley, sorghum, cassava, tapioca, potato and any combination thereof. In another aspect, the starch substrate is granular starch or liquefied starch. In a further aspect, the starch substrate is about 15% to 50%, about 15% to 30%, or about 15% to 25% dry solid (DS).

The end product may be selected from the group consisting of methanol, ethanol, butanol, monosodium glutamate, succinic acid, 1,3-propanediol, vitamins, amino acids, and lactic acid. Preferably, the end product is ethanol, 1,3-propanediol, or succinic acid.

The glucoamylase possesses at least 50% activity at pH 6.0 or above relative to its maximum activity, and is *Humicola grisea* glucoamylase (HgGA) comprising SEQ ID NO: 3, or a variant having at least 99% sequence identity. Optionally, the variant has one amino acid modifications compared to the parent glucoamylase. In another aspect, the HgGA is SEQ ID NO: 3, and is optionally produced from a *Trichoderma reesei* host cell.

In one embodiment, the glucoamylase is added at a range of about 0.1 to about 2.0, about 0.2 to about 1.0, or 0.5 to 1.0 GAU per gram of dry substance starch. In another embodiment, saccharifying further comprises adding an alpha-amylase. The alpha-amylase is from a *Bacillus* species, or a variant thereof. The alpha-amylase is a *Bacillus subtilis* alpha-amylase (AmyE), a *Bacillus amyloliquefaciens* alpha-amylase, a *Bacillus licheniformis* alpha-amylase, a *Bacillus stearothermophilus* alpha-amylase, or a variant thereof.

In another embodiment, the invention provides for SSF methods of processing starch comprising saccharifying a starch substrate to fermentable sugars at pH 6.0 to 7.5 in the presence of glucoamylase and at least one other enzyme, wherein the glucoamylase possesses at least 50% activity at pH 6.0 or above relative to its maximum activity, wherein the glucoamylase is *Humicola grisea* glucoamylase (HgGA) comprising SEQ ID NO: 3, or a variant thereof, wherein the variant has at least 99% sequence identity and wherein the other enzyme is selected from the group consisting of proteases, pullulanases, isoamylases, cellulases, hemicellulases, xylanases, cyclodextrin glycotransferases, lipases, phytases, laccases, oxidases, esterases, cutinases, xylanases, and alpha-glucosidases.

In another embodiment, the invention provides for SSF methods of processing starch comprising saccharifying a starch substrate to fermentable sugars at pH 6.0 to 7.5 in the presence of glucoamylase and at least one other non-starch polysaccharide hydrolyzing enzymes, wherein the glucoamylase possesses at least 50% activity at pH 6.0 or above relative to its maximum activity, wherein the glucoamylase is *Humicola grisea* glucoamylase (HgGA) comprising SEQ ID NO: 3, and a variant thereof, and wherein the variant has at least 99% sequence identity and wherein the non-starch polysaccharide hydrolyzing enzymes is selected from the group consisting of cellulases, hemicellulases and pectinases.

### BRIEF DESCRIPTION OF THE DRAWINGS

In the drawings:
FIG. 1 depicts the pH profiles of HgGA, TrGA, AnGA, and TeGA, at 32°C. The pH profiles are presented as the percentage of the maximum activity under the saccharification conditions described in Example 1.
FIG. 2 depicts the presence of higher sugars after 48-hour saccharification reactions catalyzed by HgGA, TrGA, and AnGA. The saccharification reactions are described in Example 4.
FIG. 3 depicts scanning electron micrographs of corn, wheat, and cassava starch treated with HgGA and an alpha-amylase at pH 6.4. Starch samples are hydrolyzed by HgGA and an alpha-amylase under the conditions as described in Example 7.

### DETAILED DESCRIPTION

The present invention relates to the use of a glucoamylase capable of effectively saccharifying a starch substrate at pH 6.0 to 7.5 in an SSF process. At a pH of 6.0 or above, the glucoamylase retains at least about 50% activity relative to the maximum activity. The glucoamylases having the unusually properties are glucoamylases from Humicola grisea, as defined in Claim 1. One embodiment is a method of using the glucoamylase to perform simultaneous saccharification and fermentation (SSF), at a neutral pH, 6.0 to 7.5, produce 1,3-propanediol, succinic acid, lysine, monosodium glutamate, or lactic acid.

In some aspects, the embodiments of the present disclosure rely on routine techniques and methods used in the field of genetic engineering and molecular biology. The following resources include descriptions of general methodology useful in accordance with the invention: Sambrook et al., MOLECULAR CLONING: A LABORATORY MANUAL (2nd Ed., 1989); Kreigler, GENE TRANSFER AND EXPRESSION; A LABORATORY MANUAL (1990) and Ausubel et al., Eds. CURRENT PROTOCOLS IN MOLECULAR BIOLOGY (1994). Unless defined otherwise herein, all technical and scientific terms used herein have the same meaning as commonly understood by one of ordinary skill in the art to which this invention belongs. Singleton, et al., DICTIONARY OF MICROBIOLOGY AND MOLECULAR BIOLOGY, 2D ED., John Wiley and Sons, New York (1994), and Hale & Markham, THE HARPER COLLINS DICTIONARY OF BIOLOGY, Harper Perennial, N.Y. (1991) provide one of skill with a general dictionary of many of the terms used herein. Although any methods and materials similar or equivalent to those described herein can be used in the practice or testing of the present invention, the representative methods and materials are described. Numeric ranges are inclusive of the numbers defining the range.

### 1. Definitions and Abbreviations

In accordance with this detailed description, the following abbreviations and definitions apply. It should be noted that as used herein, the singular forms "a," "an," and "the" include plural referents unless the context clearly dictates otherwise. Thus, for example, reference to "an enzyme" includes a plurality of such enzymes.

### 1.1. Definitions

As used herein, "amino acid sequence" is synonymous with the term "polypeptide" and/or the term "protein." In some instances, the term "amino acid sequence" is synonymous with the term "peptide"; in some instances, the term "amino acid sequence" is synonymous with the term "enzyme."

As used herein, "nucleotide sequence" or "nucleic acid sequence" refers to a sequence of genomic, synthetic, or recombinant origin and may be double-stranded or single-stranded, whether representing the sense or anti-sense strand. As used herein, the term "nucleic acid" may refer to genomic DNA, cDNA, synthetic DNA, or RNA. The residues of a nucleic acid may contain any of the chemically modifications commonly known and used in the art.

"Isolated" means that the material is at least substantially free from at least one other component that the material is naturally associated and found in nature.

"Purified" means that the material is in a relatively pure state, *e.g*., at least about 90% pure, at least about 95% pure, at least about 98% pure, or at least about 99% pure.

"Oligosaccharide" means a carbohydrate molecule composed of 3-20 mono saccharides.

As used herein, "transformed cell" includes cells that have been transformed by use of recombinant DNA techniques. Transformation typically occurs by insertion of one or more nucleotide sequences into a cell. The inserted nucleotide sequence may be a heterologous nucleotide sequence, *i.e*., is a sequence that may not be natural to the cell that is to be transformed, such as a fusion protein.

As used herein, "starch" refers to any material comprised of the complex polysaccharide carbohydrates of plants, comprised of amylose and amylopectin with the formula (C₆H₁₀O₅)ₓ, wherein "X" can be any number. In particular, the term refers to any plant-based material including but not limited to grains, grasses, tubers and roots and more specifically wheat, barley, corn, rye, rice, sorghum, brans, cassava, millet, potato, sweet potato, and tapioca.

As used herein, "granular starch" refers to uncooked (raw) starch, which has not been subject to gelatinization.

As used herein, "starch gelatinization" means solubilization of a starch molecule to form a viscous suspension.

As used herein, "gelatinization temperature" refers to the lowest temperature at which gelatinization of a starch substrate occurs. The exact temperature depends upon the specific starch substrate and further may depend on the particular variety and the growth conditions of plant species from which the starch is obtained.

"DE" or "dextrose equivalent" is an industry standard for measuring the concentration of total reducing sugars, calculated as the percentage of the total solids that have been converted to reducing sugars. The granular starch that has not been hydrolyzed has a DE that is about zero (0), and D-glucose has a DE of about 100.

As used herein, "starch substrate" refers to granular starch or liquefied starch using refined starch, whole ground grains, or fractionated grains.

As used herein, "liquefied starch" refers to starch that has gone through solubilization process, for example, the conventional starch liquefaction process.

As used herein, "glucose syrup" refers to an aqueous composition containing glucose solids. Glucose syrup will have a DE of at least about 20. In some embodiments, glucose syrup may contain no more than about 21% water while at least about 25% reducing sugar calculated as dextrose. Glucose syrup may include at least about 90% D-glucose, or at least about 95% D-glucose. The terms glucose and glucose syrup are used interchangeably.

"Degree of polymerization (DP)" refers to the number (n) of anhydroglucopyranose units in a given saccharide. Examples of DP1 are the monosaccharides, such as glucose and fructose. Examples of DP2 are the disaccharides, such as maltose and sucrose. A DP4+ (>DP4) denotes polymers with a degree of polymerization of greater than four.

As used herein, "fermentable sugars" refer to saccharides that are capable of being metabolized under fermentation conditions. These sugars typically refer to glucose, maltose, and maltotriose (DP1, DP2 and DP3).

As used herein, "total sugar content" refers to the total sugar content present in a starch composition.

As used herein, "ds" refers to dissolved solids in a solution. The term "dry solids content (DS)" refers to the total solids of a slurry in % on a dry weight basis. The term "slurry" refers to an aqueous mixture containing insoluble solids.

As used herein, "starch-liquefying enzyme" refers to an enzyme that catalyzes the hydrolysis or breakdown of granular starch. Exemplary starch liquefying enzymes include alpha-amylases (EC 3.2.1.1).

"Amylase" means an enzyme that is, among other things, capable of catalyzing the degradation of starch. For example, β-Amylases, α-glucosidases (EC 3.2.1.20; α-D-glucoside glucohydrolase), glucoamylase (EC 3.2.1.3; α-D-(1→4)-glucan glucohydrolase), and product-specific amylases can produce malto-oligosaccharides of a specific length from starch.

"Alpha-amylases (EC 3.2.1.1)" refer to endo-acting enzymes that cleave α-D-(1→4) O-glycosidic linkages within the starch molecule in a random fashion. In contrast, the exo-acting amylolytic enzymes, such as beta-amylases (EC 3.2.1.2; α-D-(1→4)-glucan maltohydrolase) and some product-specific amylases like maltogenic alpha-amylase (EC 3.2.1.133) cleave the starch molecule from the non-reducing end of the substrate. These enzymes have also been described as those effecting the exo- or endohydrolysis of 1,4-α-D-glucosidic linkages in polysaccharides containing 1, 4-α-linked D-glucose units. Another term used to describe these enzymes is glycogenase. Exemplary enzymes include alpha-1, 4-glucan 4-glucanohydrolase.

As used herein, "glucoamylases" refer to the amyloglucosidase class of enzymes (EC 3.2.1.3, glucoamylase, α-1, 4-D-glucan glucohydrolase). These are exo-acting enzymes that release glucosyl residues from the non-reducing ends of amylose and/or amylopectin molecules. The enzymes are also capably of hydrolyzing α-1, 6 and α-1,3 linkages, however, at much slower rates than the hydrolysis of α-1, 4 linkages.

As used herein, the term "non-starch polysaccharide hydrolyzing enzymes" are enzymes capable of hydrolyzing complex carbohydrate polymers such as cellulose, hemicellulose, and pectin. For example, cellulases (endo and exo-glucanases, beta glucosidase) hemicellulases (xylanases) and pectinases are non-starch polysaccharide hydrolyzing enzymes.

As used herein, "maximum activity" refers to the enzyme activity measured under the most favorable conditions, for example, at an optimum pH. As used herein, "optimum pH" refers to a pH value, under which the enzyme displays the highest activity with other conditions being equal.

The phrase "mature form" of a protein or polypeptide refers to the final functional form of the protein or polypeptide. A mature form of a glucoamylase may lack a signal peptide and/or initiator methionine, for example. A mature form of a glucoamylase may be produced from its native host, for example, by endogenous expression. Alternatively, a mature form of a glucoamylase may be produced from a non-native host, for example, by exogenous expression. An exogenously expressed glucoamylase, while maintaining the glucoamylase activity, may have a varied glycosylation pattern compared to the endogenous expressed counterpart.

The term "parent" or "parent sequence" refers to a sequence that is native or naturally occurring in a host cell.

As used herein, the terms "variant" is used in reference to glucoamylases that have some degree of amino acid sequence identity to a parent glucoamylase sequence. A variant is similar to a parent sequence, but has at least one substitution, deletion or insertion in their amino acid sequence that makes them different in sequence from a parent glucoamylase. In some cases, variants have been manipulated and/or engineered to include at least one substitution, deletion, or insertion in their amino acid sequence that makes them different in sequence from a parent. Additionally, a glucoamylase variant may retain the functional characteristics of the parent glucoamylase, e.g., maintaining a glucoamylase activity that is at least 50%, about 60%, about 70%, about 80%, or about 90% of that of the parent glucoamylase.

The variants for use in the present invention have at least 99% sequence identity with SEQ ID NO:3.

As used herein, "hydrolysis of starch" refers to the cleavage of glucosidic bonds with the addition of water molecules.

As used herein, "no-cook" refers to a process of converting a granular starch substrate, for example, raw starch, to fermentable sugars without the conventional high-temperature starch liquefaction process.

As used herein, "end product" or "desired end product" refers to a molecule or compound to which a starch substrate is converted into, by an enzyme and/or a microorganism.

As used herein, "contacting" or "admixing" refers to the placing of the respective enzyme(s) in sufficiently close proximity to the respective substrate to enable the enzyme(s) to convert the substrate to the end product. Those skilled in the art will recognize that mixing solutions of the enzyme with the respective substrates can affect contacting or admixing.

### 1.2. Abbreviations

The following abbreviations apply unless indicated otherwise:
- AkAA: *Aspergillus kawachii* alpha-amylase
- AmyE: *Bacillus subtilis* alpha-amylase
- AmyL: *Bacillus licheniformis* alpha-amylase
- AmyR: SPEZYME® XTRA amylase
- AmyS: *Geobacillus stearothermophilus* alpha-amylase
- AnGA: *Aspergillus niger* glucoamylase
- BAA: bacterial alpha-amylase
- cDNA: complementary DNA
- DE: Dextrose Equivalent
- DI: distilled, deionized
- DNA: deoxyribonucleic acid
- DP3: degree of polymerization with three subunits
- DPn: degree of polymerization with n subunits
- DS or ds: dry solid
- dss: dry solid starch
- EC: enzyme commission for enzyme classification
- g: gram
- gpm: gallon per minute
- GAU: glucoamylase units
- HGA: *Humicola grisea* glucoamylase
- HgGA: *Humicola grisea* glucoamylase
- HPLC: high pressure liquid chromatography
- kg: kilogram
- MOPS: 3-(N-morpholino)propanesulfonic acid
- MT: metric ton
- MW: molecular weight
- NCBI: National Center for Biotechnology Information
- nm: nanometer
- OD: optical density
- PCR: polymerase chain reaction
- PEG: polyethylene glycol
- pI: isoelectric point
- ppm: parts per million
- RhGA: *Rhizopus* sp. glucoamylase
- RNA: ribonucleic acid
- RO: reverse osmosis
- rpm: revolutions per minute
- slpm: standard liters per minute
- SSF: simultaneous saccharification and fermentation
- TeGA: *Talaromyces emersonii* glucoamylase
- TrGA: *Trichoderma reesei* glucoamylase
- w/v: weight/volume
- w/w: weight/weight
- wt: wild-type
- µL: microliter

### 2. Enzymes in Starch Processing

### 2.1. Glucoamylase

### 2.1.1. Structure and Function

Glucoamylases are produced by numerous strains of bacteria, fungi, yeast and plants. Many fungal glucoamylases are fungal enzymes that are extracellularly produced, for example from strains of *Aspergillus* (Svensson et al., Carlsberg Res. Commun. 48: 529-544 (1983); Boel et al., EMBO J. 3: 1097-1102 (1984); Hayashida et al., Agric. Biol. Chem. 53: 923-929 (1989); U.S. Patent No. 5,024,941; U.S. Patent No. 4,794,175 and WO 88/09795); *Talaromyces* (U.S. Patent No. 4,247,637; U.S. Patent No. 6,255,084; and U.S. Patent No. 6,620,924); *Rhizopus* (Ashikari et al., Agric. Biol. Chem. 50: 957-964 (1986); Ashikari et al., App. Microbio. Biotech. 32: 129-133 (1989) and U.S. Patent No. 4,863,864); *Humicola* (WO 05/052148 and U.S. Patent No. 4,618,579); and *Mucor* (Houghton-Larsen et al., Appl. Microbiol. Biotechnol. 62: 210-217 (2003)). Many of the genes that code for these enzymes have been cloned and expressed in yeast, fungal and/or bacterial cells.

Commercially, glucoamylases are very important enzymes and have been used in a wide variety of applications that require the hydrolysis of starch (*e.g*., for producing glucose and other monosaccharides from starch). Glucoamylases are used to produce high fructose corn sweeteners, which comprise over 50% of the sweetener market in the United States. In general, glucoamylases may be, and commonly are, used with alpha-amylases in starch hydrolyzing processes to hydrolyze starch to dextrins and then glucose. The glucose may then be converted to fructose by other enzymes (*e.g*., glucose isomerases); crystallized; or used in fermentations to produce numerous end products (*e.g*., ethanol, citric acid, succinic acid, ascorbic acid intermediates, glutamic acid, glycerol, 1,3-propanediol and lactic acid).

Glucoamylases consist of as many as three distinct structural domains, a catalytic domain of approximately 450 residues that is structurally conserved in all glucoamylases, generally followed by a linker region consisting of between 30 and 80 residues that are connected to a starch binding domain of approximately 100 residues. The structure of the *Trichoderma reesei* glucoamylase (TrGA) with all three regions intact was determined to 1.8 Angstrom resolution. *See* WO 2009/048488 and WO 2009/048487. Using the determined coordinates, the structure was aligned with the coordinates of the catalytic domain of the glucoamylase from *Aspergillus awamori* strain X100 that was determined previously (Aleshin, A.E., Hoffman, C., Firsov, L.M., and Honzatko, R.B. Refined crystal structures of glucoamylase from Aspergillus awamori var. X100. J. Mol. Biol. 238: 575-591 (1994)). *See id.* The structure of the catalytic domains of these two glucoamylases overlap very closely, and it is possible to identify equivalent residues based on this structural superposition. *See id.* It is further believed that all glucoamylases share the basic structure. *See id.*

Given the well-known structure and function relationship of glucoamylases, glucoamylase variants having altered properties have been successfully created and characterized. The variants may display improved properties as compared to the parent glucoamylases. The improved properties may include and are not limited to increased thermostability and increased specific activity. For example, methods for making and characterizing TrGA variants with altered properties have been described in WO 2009/067218.

### 2.1.2. Glucoamylases having the desired pH profile

The embodiments of the present disclosure utilize a Humcola grisea glucoamylase (HgGA), capable of effectively saccharifying a starch substrate at a neutral pH, between pH 6.0 and 7.5. At a pH of 6.0 or above, the glucoamylase retains at least 50%, about 51%, about 52%, about 53%, about 54%, or about 55% of the activity relative to the maximum activity.

HgGA is defined in Claim 1 and may be the glucoamylase comprising the amino acid sequence of SEQ ID NO: 3, which is described in detail in U.S. Patent Nos. 4,618,579 and 7,262,041. This HgGA is also described as a granular starch hydrolyzing enzyme (GSHE), because it is capable of hydrolyzing starch in granular form. The genomic sequence coding the HgGA from *Humicola grisea* var. *thermoidea* is presented as SEQ ID NO: 1, which contains three putative introns (positions 233-307, 752-817, and 950-1006). The native HgGA from *Humicola grisea* var. *thermoidea* has the amino acid sequence of SEQ ID NO: 2, which includes a signal peptide containing 30 amino acid residues (positions 1 to 30 of SEQ ID NO: 2). Cleavage of the signal peptide results in the mature HgGA having the amino acid sequence of SEQ ID NO: 3. The HgGA may be produced from a *Trichoderma* host cell, *e.g*., a *Trichoderma reesei* cell. *See* U.S. Patent No 7,262,041.

The glucoamylase for use in the present invention may also be a variant of HgGA. The variant has at least 99% sequence identity to the parent glucoamylase. Optionally, the variant has one, two, three, four, five, or six amino acids modification compared to the mature form of the parent glucoamylase. The variant possesses the desired pH profile and capability of saccharifying a starch substrate at a pH in the range of 6.0 to 7.5. The variants may possess other improved properties, such as improved thermostability and improved specificity.

### 2.1.3. Production of Glucoamylase

Glucoamylases may be produced with recombinant DNA technology in various host cells.

The host cells can be selected from bacterial, fungal, plant and yeast cells. The term host cell includes both the cells, progeny of the cells and protoplasts created from the cells that are used to produce a variant glucoamylase according to the disclosure. The host cells can be fungal cells and typically filamentous fungal host cells. The term "filamentous fungi" refers to all filamentous forms of the subdivision Eumycotina (*See*, Alexopoulos, C. J. (1962), INTRODUCTORY MYCOLOGY, Wiley, New York). These fungi are characterized by a vegetative mycelium with a cell wall composed of chitin, cellulose, and other complex polysaccharides. The filamentous fungi are morphologically, physiologically, and genetically distinct from yeasts. Vegetative growth by filamentous fungi is by hyphal elongation and carbon catabolism is obligatory aerobic. The filamentous fungal parent cell may be a cell of a species of, but not limited to, *Trichoderma,* (*e.g*., *Trichoderma reesei,* the asexual morph of *Hypocrea jecorina*, previously classified as *T. longibrachiatum, Trichoderma viride, Trichoderma koningii, Trichoderma harzianum)* (Sheir-Neirs et al., (1984) Appl. Microbiol. Biotechnol 20:46-53; ATCC No. 56765 and ATCC No. 26921); *Penicillium* sp., *Humicola* sp. (*e.g*., *H. insolens, H. lanuginosa and H. grisea*)*; Chrysosporium* sp. (*e.g., C. lucknowense), Gliocladium* sp., *Aspergillus* sp. (*e.g*., *A. oryzae, A. niger, A sojae, A. japonicus, A. nidulans,* and A. *awamori*) (Ward et al., (1993) Appl. Microbiol. Biotechnol. 39:738-743 and Goedegebuur et al., (2002) Genet 41:89-98), *Fusarium sp.,(e.g., F. roseum, F. graminum F. cerealis, F. oxysporuim* and *F. venenatum), Neurospora* sp., *(N. crassa), Hypocrea* sp., *Mucor sp.,(M. miehei*),, *Rhizopus* sp. *and Emericella* sp. (*see also,* Innis et al., (1985) Sci. 228:21-26). The term "*Trichoderma*" or "*Trichoderma* sp." or "*Trichoderma* spp." refers to any fungal genus previously or currently classified as *Trichoderma.* The host cell will be a genetically engineered host cell wherein native genes have been inactivated, for example by deletion in fungal cells. Where it is desired to obtain a fungal host cell having one or more inactivated genes known methods may be used (e.g. methods disclosed in U.S. Patent Nos. 5,246,853 and 5,475,101, and WO 92/06209). Gene inactivation may be accomplished by complete or partial deletion, by insertional inactivation or by any other means that renders a gene nonfunctional for its intended purpose (such that the gene is prevented from expression of a functional protein). When the host cell is a *Trichoderma* cell and particularly a *T. reesei* host cell, the *cbh*1, *cbh*2, *egl*1 and *egl*2 genes will be inactivated and/or typically deleted. Typically, *Trichoderma reesei* host cells having quad-deleted proteins are set forth and described in U.S. Patent No. 5,847,276 and WO 05/001036. The host cell can be a protease deficient or protease minus strain.

To produce the glucoamylase as defined in Claim 1 with recombinant DNA technology, a DNA construct comprising nucleic acid encoding the amino acid sequence of the designated glucoamylase can be constructed and transferred into, for example, a *Trichoderma reesei* host cell. The vector may be any vector which when introduced into a *Trichoderma reesei* host cell can be integrated into the host cell genome and can be replicated. Reference is made to the Fungal Genetics Stock Center Catalogue of Strains (FGSC, <www.fgsc.net>) for a list of vectors. Additional examples of suitable expression and/or integration vectors are provided in Sambrook et al., (1989) supra, and Ausubel (1987) supra, and van den Hondel et al. (1991) in Bennett and Lasure (Eds.) MORE GENE MANIPULATIONS IN FUNGI, Academic Press pp. 396 428 and U.S. Patent No. 5,874,276. The nucleic acid encoding the glucoamylase can be operably linked to a suitable promoter, which shows transcriptional activity in *Trichoderma reesei* host cell. The promoter may be derived from genes encoding proteins either homologous or heterologous to the host cell. Suitable non-limiting examples of promoters include *cbh1*, *cbh2*, *egl1*, *egl2.* The promoter may be a native *T. reesei* promoter. Typically, the promoter can be *T. reesei cbh1*, which is an inducible promoter and has been deposited in GenBank under Accession No. D86235. An "inducible promoter" may refer to a promoter that is active under environmental or developmental regulation. The promoter can be one that is heterologous to *T. reesei* host cell. Other examples of useful promoters include promoters from A. *awamori* and A. *niger* glucoamylase genes (*see, e.g*., Nunberg et al., (1984) Mol. Cell Biol. 4:2306-2315 and Boel et al., (1984) EMBO J. 3:1581-1585). Also, the promoters of the *T. reesei xln1* gene and the cellobiohydrolase 1 gene may be useful (EPA 13f280A1).

The glucoamylase coding sequence can be operably linked to a signal sequence. The signal sequence may be the native signal peptide of the glucoamylase (residues 1-20 of SEQ ID NO: 2 for HgGA, or residues 1-33 of SEQ ID NO: 5 for TrGA, for example). Alternatively, the signal sequence may have at least 90% or at least 95% sequence identity to the native signal sequence. A signal sequence and a promoter sequence comprising a DNA construct or vector to be introduced into the *T. reesei* host cell can be derived from the same source. For example, the signal sequence can be the *cdhl* signal sequence that is operably linked to a *cdhl* promoter.

The expression vector may also include a termination sequence. The termination sequence and the promoter sequence can be derived from the same source. The termination sequence can be homologous to the host cell. A particularly suitable terminator sequence can be *cbh1* derived from *T. reesei.* Other exemplary fungal terminators include the terminator from A. *niger* or A. *awamori* glucoamylase gene.

An vector may include a selectable marker. Examples of representative selectable markers include ones that confer antimicrobial resistance (e.g., hygromycin and phleomycin). Nutritional selective markers also find use, including those markers known in the art as *amdS, argB,* and *pyr4.* Markers useful in vector systems for transformation of *Trichoderma* are known in the art *(see,* e.g., Finkelstein, chapter 6 in BIOTECHNOLOGY OF FILAMENTOUS FUNGI, Finkelstein et al. Eds. Butterworth-Heinemann, Boston, Mass. (1992), Chap. 6.; and Kinghorn et al. (1992) APPLIED MOLECULAR GENETICS OF FILAMENTOUS FUNGI, Blackie Academic and Professional, Chapman and Hall, London). The selective marker may be the *amdS* gene, which encodes the enzyme acetamidase, allowing transformed cells to grow on acetamide as a nitrogen source. The use of A. *nidulans amdS* gene as a selective marker is described for example in Kelley et al., (1985) EMBO J. 4:475-479 and Penttila et al., (1987) Gene 61:155-164.

An expression vector comprising a DNA construct with a polynucleotide encoding the glucoamylase may be any vector which is capable of replicating autonomously in a given fungal host organism or of integrating into the DNA of the host. The expression vector can be a plasmid. Typically, two types of expression vectors for obtaining expression of genes are contemplated.

The first expression vector may comprise DNA sequences in which the promoter, glucoamylase-coding region, and terminator all originate from the gene to be expressed. Gene truncation can be obtained by deleting undesired DNA sequences (e.g., DNA encoding unwanted domains) to leave the domain to be expressed under control of its own transcriptional and translational regulatory sequences.

The second type of expression vector may be preassembled and contains sequences needed for high-level transcription and a selectable marker. The coding region for the glucoamylase gene or part thereof can be inserted into this general-purpose expression vector such that it is under the transcriptional control of the expression construct promoter and terminator sequences. Genes or part thereof may be inserted downstream of a strong promoter, such as the strong *cbh1* promoter.

Methods used to ligate the DNA construct comprising a polynucleotide encoding the glucoamylase, a promoter, a terminator and other sequences and to insert them into a suitable vector are well known in the art. Linking can be generally accomplished by ligation at convenient restriction sites. If such sites do not exist, the synthetic oligonucleotide linkers are used in accordance with conventional practice. (*see*, Sambrook (1989) *supra*, and Bennett and Lasure, MORE GENE MANIPULATIONS IN FUNGI, Academic Press, San Diego (1991) pp 70-76.). Additionally, vectors can be constructed using known recombination techniques (e.g., Invitrogen Life Technologies, Gateway Technology).

Introduction of a DNA construct or vector into a host cell includes techniques such as transformation; electroporation; nuclear microinjection; transduction; transfection, (e.g., lipofection mediated and DEAE-Dextrin mediated transfection); incubation with calcium phosphate DNA precipitate; high velocity bombardment with DNA-coated microprojectiles; and protoplast fusion. General transformation techniques are known in the art *(see,* e.g., Ausubel et al., (1987), *supra*, chapter 9; and Sambrook (1989) *supra*, and Campbell et al., (1989) Curr. Genet. 16:53-56). The expression of heterologous protein in *Trichoderma* is described in U.S. Pat. Nos. 6,022,725; 6,268,328; Harkki et al. (1991); Enzyme Microb. Technol. 13:227-233; Harkki et al., (1989) Bio Technol. 7:596-603; EP 244,234; EP 215,594; and Nevalainen et al., "The Molecular Biology of Trichoderma and its Application to the Expression of Both Homologous and Heterologous Genes," in MOLECULAR INDUSTRIAL MYCOLOGY, Eds. Leong and Berka, Marcel Dekker Inc., NY (1992) pp. 129-148).

Genetically stable transformants can be constructed with vector systems whereby the nucleic acid encoding glucoamylase is stably integrated into a host strain chromosome. Transformants are then purified by known techniques.

In one non-limiting example, stable transformants including an *amdS* marker are distinguished from unstable transformants by their faster growth rate and the formation of circular colonies with a smooth, rather than ragged outline on solid culture medium containing acetamide. Additionally, in some cases a further test of stability can be conducted by growing the transformants on solid non-selective medium (i.e., medium that lacks acetamide), harvesting spores from this culture medium and determining the percentage of these spores which subsequently germinate and grow on selective medium containing acetamide. Alternatively, other methods known in the art may be used to select transformants.

Uptake of DNA into the host *Trichoderma* sp. strain is dependent upon the calcium ion concentration. Generally, between about 10 mM CaCl₂ and 50 mM CaCl₂ may be used in an uptake solution. Besides the need for the calcium ion in the uptake solution, other compounds generally included are a buffering system such as TE buffer (10 mM Tris, pH 7.4; 1 mM EDTA) or 10 mM MOPS, pH 6.0 buffer (morpholinepropanesulfonic acid) and polyethylene glycol (PEG). It is believed that the polyethylene glycol acts to fuse the cell membranes, thus permitting the contents of the medium to be delivered into the cytoplasm of the *Trichoderma* sp. strain and the plasmid DNA is transferred to the nucleus. This fusion frequently leaves multiple copies of the plasmid DNA integrated into the host chromosome.

Usually a suspension containing the *Trichoderma* sp. protoplasts or cells that have been subjected to a permeability treatment at a density of 10⁵ to 10⁷/mL, typically, 2 × 10⁶/mL are used in transformation. A volume of 100 µL of these protoplasts or cells in an appropriate solution (e.g., 1.2 M sorbitol; 50 mM CaCl₂) are mixed with the desired DNA. Generally, a high concentration of PEG may be added to the uptake solution. From 0.1 to 1 volume of 25% PEG 4000 can be added to the protoplast suspension. It is also typical to add about 0.25 volumes to the protoplast suspension. Additives such as dimethyl sulfoxide, heparin, spermidine, potassium chloride and the like may also be added to the uptake solution and aid in transformation. Similar procedures are available for other fungal host cells. *See,* e.g., U.S. Pat. Nos. 6,022,725 and 6,268,328.

Generally, the mixture can be then incubated at approximately 0°C for a period of between 10 to 30 minutes. Additional PEG may then be added to the mixture to further enhance the uptake of the desired gene or DNA sequence. The 25% PEG 4000 can be generally added in volumes of 5 to 15 times the volume of the transformation mixture; however, greater and lesser volumes may be suitable. The 25% PEG 4000 may be typically about 10 times the volume of the transformation mixture. After the PEG is added, the transformation mixture can then be incubated either at room temperature or on ice before the addition of a sorbitol and CaCl₂ solution. The protoplast suspension can then be further added to molten aliquots of a growth medium. This growth medium permits the growth of transformants only.

Generally, cells are cultured in a standard medium containing physiological salts and nutrients (*see*, e.g., Pourquie, J. et al., BIOCHEMISTRY AND GENETICS OF CELLULOSE DEGRADATION, eds. Aubert, J. P. et al., Academic Press, pp. 71 86, 1988 and IImen, M. et al., (1997) Appl. Environ. Microbiol. 63:1298-1306). Common commercially prepared media (e.g., Yeast Malt Extract (YM) broth, Luria Bertani (LB) broth and Sabouraud Dextrose (SD) broth also find use in the present embodiments.

Culture-conditions are also standard, (e.g., cultures are incubated at approximately 28°C in appropriate medium in shake cultures or fermentors until desired levels of glucoamylase expression are achieved). After fungal growth has been established, the cells are exposed to conditions effective to cause or permit the expression of the glucoamylase. In cases where the glucoamylase coding sequence is under the control of an inducible promoter, the inducing agent (e.g., a sugar, metal salt or antimicrobial), can be added to the medium at a concentration effective to induce glucoamylase expression.

In general, the glucoamylase produced in cell culture may be secreted into the medium and may be purified or isolated, e.g., by removing unwanted components from the cell culture medium. In some cases, the glucoamylase can be produced in a cellular form, necessitating recovery from a cell lysate. In such cases, the enzyme may be purified from the cells in which it was produced using techniques routinely employed by those of skill in the art. Examples of these techniques include, but are not limited to, affinity chromatography (Tilbeurgh et a., (1984) FEBS Lett. 16: 215), ion-exchange chromatographic methods (Goyal et al., (1991) Biores. Technol. 36: 37; Fliess et al., (1983) Eur. J. Appl. Microbiol. Biotechnol. 17: 314; Bhikhabhai et al, (1984) J. Appl. Biochem. 6: 336; and Ellouz et al., (1987) Chromatography 396: 307), including ion-exchange using materials with high resolution power (Medve et al., (1998) J. Chromatography A 808: 153), hydrophobic interaction chromatography (see, Tomaz and Queiroz, (1999) J. Chromatography A 865: 123; two-phase partitioning (*see*, Brumbauer, et al., (1999) Bioseparation 7: 287); ethanol precipitation; reverse phase HPLC, chromatography on silica or on a cation-exchange resin such as DEAE, chromatofocusing, SDS-PAGE, ammonium sulfate precipitation, and gel filtration (e.g., Sephadex G-75).

### 2.2. Alpha-amylases

Alpha-amylases constitute a group of enzymes present in microorganisms and tissues from animals and plants. They are capable of hydrolyzing alpha-1,4-glucosidic bonds of glycogen, starch, related polysaccharides, and some oligosaccharides. Although all alpha-amylases possess the same catalytic function, their amino acid sequences vary greatly. The sequence identity between different amylases can be virtually non-existent, e.g., falling below 25%. Despite considerable amino acid sequence variation, alpha-amylases share a common overall topological scheme that has been identified after the three-dimensional structures of alpha-amylases from different species have been determined. The common three-dimensional structure reveals three domains: (1) a "TIM" barrel known as domain A, (2) a long loop region known as domain B that is inserted within domain A, and (3) a region close to the C-terminus known as domain C that contains a characteristic beta-structure with a Greek-key motif.

"Termamyl-like" alpha-amylases refer to a group of alpha-amylases widely used in the starch-processing industry. The *Bacillus licheniformis* alpha-amylase having an amino acid sequence of SEQ ID NO: 2 of U.S. Patent No. 6,440,716 is commercially available as Termamyl®. Termamyl-like alpha-amylases commonly refer to a group of highly homologous alpha-amylases produced by *Bacillus* spp. Other members of the group include the alpha-amylases from *Geobacillus stearothermophilus* (previously known as *Bacillus stearothermophilus*; both names are used interchangeably in the present disclosure) and *Bacillus amyloliquefaciens*, and those derived from *Bacillus* sp. NCIB 12289, NCIB 12512, NCIB 12513, and DSM 9375, all of which are described in detail in U.S. Patent No. 6,440,716 and WO 95/26397.

Although alpha-amylases universally contain the three domains discussed above, the three-dimensional structures of some alpha-amylases, such as AmyE from *Bacillus subtilis*, differ from Termamyl-like alpha-amylases. These enzymes are collectively referred as non-Termamyl-like alpha-amylases. "AmyE" for the purpose of this disclosure means a naturally occurring alpha-amylase (EC 3.2.1.1; 1, 4-α-D-glucan glucanohydrolase) from *Bacillus subtilis.* Representative AmyE enzymes and the variants thereof are disclosed in U.S. Patent Application 12/478,266 (US 2009/0305935) and 12/478,368 (US 2009/0305360), both filed June 4, 2009, and 12/479,427 (US 2010/0015686), filed June 5,2009.

Other commercially available amylases can be used, e.g., TERMAMYL^{®} 120-L, LC and SC SAN SUPER^{®}, SUPRA^{®}, and LIQUEZYME^{®} SC available from Novo Nordisk A/S, FUELZYME^{®} FL from Diversa, and CLARASE^{®} L, SPEZYME^{®} FRED, SPEZYME^{®} ETHYL, GC626, and GZYME^{®} G997 available from Danisco, US, Inc., Genencor Division.

### 2.3. Other enzymes and enzyme combinations

In embodiments of the present disclosure, other enzyme(s) may also be supplemented in the SSF process. These supplementary enzymes may include proteases, pullulanases, isoamylases, cellulases, hemicellulases, xylanases, cyclodextrin glycotransferases, lipases, phytases, laccases, oxidases, esterases, cutinases, xylanases, and/or alpha-glucosidases. *See e.g*., WO 2009/099783. Skilled artisans in the art are well aware of the methods using the above-listed enzymes.

The glucoamylases, as defined in Claim 1, can be used in combination with any other enzyme. For example, glucoamylase maybe used in combination with amylases (e.g., alpha-amylases). In one embodiment, the simultaneous saccharification and fermentation (SSF) process use glucoamylase and one or more non-starch polysaccharide hydrolyzing enzymes. These enzymes are capable of hydrolyzing complex carbohydrate polymers such as cellulose, hemicellulose, and pectin. Non-limiting examples include cellulases (e.g., endo and exo-glucanases, beta glucosidase) hemicellulases (e.g., xylanases) and pectinases. In another embodiment, the SSF process use glucoamylase, alpha-amylase and one or more non-starch polysaccharide hydrolyzing enzymes. In another embodiment, the SSF process use glucoamylase with phytases, proteases, isoamylases and pullulanases.

In some embodiments, the SSF process can use at least two non-starch polysaccharide hydrolyzing enzymes. In some embodiments, the SSF process can use at least three non-starch polysaccharide hydrolyzing enzymes.

Cellulases are enzyme compositions that hydrolyze cellulose (β-1,4-D-glucan linkages) and/or derivatives thereof, such as phosphoric acid swollen cellulose. Cellulases include the classification of exo-cellobiohydrolases (CBH), endoglucanases (EG) and β-glucosidases (BG) (EC3.2.191, EC3.2.1.4 and EC3.2.1.21). Examples of cellulases include cellulases from Penicillium, Trichoderma, Humicola, Fusarium, Thermomonospora, Cellulomonas, Hypocrea, Clostridium, Thermomonospore, Bacillus, Cellulomonas and Aspergillus. Non-limiting examples of commercially available cellulases sold for feed applications are beta-glucanases such as ROVABIO^{®} (Adisseo), NATUGRAIN^{®} (BASF), MULTIFECT^{®} BGL (Danisco Genencor) and ECONASE^{®} (AB Enzymes). Some commercial cellulases includes ACCELERASE^{®}. The cellulases and endoglucanases described in US20060193897A1 also may be used.

Beta-glucosidases (cellobiase) hydrolyzes cellobiose into individual monosaccharides. Various beta glucanases find use in the invention in combination with phytases. Beta glucanases (endo-cellulase-enzyme classification EC 3.2.1.4) also called endoglucanase I, II, and III, are enzymes that will attack the cellulose fiber to liberate smaller fragments of cellulose which is further attacked by exo-cellulase to liberate glucose. Commercial beta-glucanases useful in the methods of the invention include OPTIMASH^{®} BG and OPTIMASH^{®} TBG (Danisco, US, Inc. Genencor Division).

Hemicellulases are enzymes that break down hemicellulose. Hemicellulose categorizes a wide variety of polysaccharides that are more complex than sugars and less complex than cellulose, that are found in plant walls. In some embodiments, a xylanase find use as a secondary enzyme in the methods of the invention. Any suitable xylanase can be used in the invention. Xylanases (e.g. endo-β-xylanases (E.C. 3.2.1.8), which hydrolyze the xylan backbone chain, can be from bacterial sources (e.g., Bacillus, Streptomyces, Clostridium, Acidothermus, Microtetrapsora or Thermonospora) or from fungal sources (Aspergillus, Trichoderma, Neurospora, Humicola, Penicillium or Fusarium (See, e.g., EP473 545; U.S. Pat. No. 5,612,055; WO 92/06209; and WO 97/20920)). Xylanases useful in the invention include commercial preparations (e.g., MULTIFECT^{®} and FEEDTREAT^{®} Y5 (Danisco Genencor), RONOZYME^{®} WX (Novozymes A/S) and NATUGRAIN WHEAT^{®} (BASF). In some embodiments the xylanase is from Trichoderma reesei or a variant xylanase from Trichoderma reesei, or the inherently thermostable xylanase described in EP1222256B1, as well as other xylanases from *Aspergillus niger, Aspergillus kawachii, Aspergillus tubigensis, Bacillus circulans, Bacillus pumilus, Bacillus subtilis, Neocallimastix patriciarum, Penicillium species, Streptomyces lividans, Streptomyces thermoviolaceus, Thermomonospora fusca, Trichoderma harzianum, Trichoderma reesei,* and *Trichoderma viridae.*

Phytases that can be used include those enzymes capable of liberating at least one inorganic phosphate from inositol hexaphosphate. Phytases are grouped according to their preference for a specific position of the phosphate ester group on the phytate molecule at which hydrolysis is initiated, (e.g., as 3-phytases (EC 3.1.3.8) or as 6-phytases (EC 3.1.3.26)). A typical example of phytase is myo-inositol-hexakiphosphate-3-phosphohydrolase. Phytases can be obtained from microorganisms such as fungal and bacterial organisms (e.g. Aspergillus (e.g., A. niger, A. terreus, and A. fumigatus), Myceliophthora (M. thermophila), Talaromyces (T. thermophilus) Trichoderma spp (T. reesei), and Thermomyces (see e.g., WO 99/49740)). Also phytases are available from Penicillium species, (e.g., P. hordei (See e.g., ATCC No. 22053), P. piceum (See e.g., ATCC No. 10519), or P. brevi-compactum (See e.g., ATCC No. 48944) (See, e.g. U.S. Pat. No. 6,475,762). Additional phytases that find use in the invention are available from Peniophora, E. coli, Citrobacter, Enterbacter and Buttiauxella (see e.g., WO2006/043178, filed Oct. 17, 2005). Additional phytases useful in the invention can be obtained commercially (e.g. NATUPHOS^{®} (BASF), RONOZYME^{®} P (Novozymes A/S), PHZYME^{®} (Danisco A/S, Diversa) and FINASE^{®} (AB Enzymes).

Various acid fungal proteases (AFP) can be used as part of the combination as well. Acid fungal proteases include for example, those obtained from *Aspergillus, Trichoderma, Mucor* and *Rhizopus,* such as *A. niger, A. awamori, A. oryzae* and *M. miehei.* AFP can be derived from heterologous or endogenous protein expression of bacteria, plants and fungi sources. IAFP secreted from strains of Trichoderma can be used. Suitable AFP includes naturally occurring wild-type AFP as well as variant and genetically engineered mutant AFP. Some commercial AFP enzymes useful in the invention include FERMGEN^{®} (Danisco US, Inc, Genencor Division), and FORMASE^{®} 200.

Proteases can also be used with glucoamylase and any other enzyme combination. Any suitable protease can be used. Proteases can be derived from bacterial or fungal sources. Sources of bacterial proteases include proteases from *Bacillus* (e.g., *B. amyloliquefaciens, B. lentus, B. licheniformis,* and *B. subtilis*). Exemplary proteases include, but are not limited to, subtilisin such as a subtilisin obtainable from *B. amyloliquefaciens* and mutants thereof (U.S. Pat. No. 4,760,025). Suitable commercial protease includes MULTIFECT^{®} P 3000 (Danisco Genencor) and SUMIZYME^{®} FP (Shin Nihon). Sources of suitable fungal proteases include, but are not limited to, *Trichoderma, Aspergillus, Humicola* and *Penicillium*, for example.

Debranching enzymes, such as an isoamylase (EC 3.2.1.68) or pullulanase (EC 3.2.1.41), can also be used in combination with the glucoamylases in the SSF processes of the invention. A non-limiting example of a pullulanase that can be used is Promozyme^{®}.

### 3. Starch Processing

### 3.1. Starch Substrates and Raw Materials

Those of skill in the art are well aware of available methods that may be used to prepare starch substrates for use in the processes disclosed herein. For example, a useful starch substrate may be obtained from tubers, roots, stems, legumes, cereals, or whole grain. More specifically, the granular starch comes from plants that produce high amounts of starch. For example, granular starch may be obtained from corn, wheat, barley, rye, milo, sago, cassava, tapioca, sorghum, rice, peas, bean, banana, or potatoes. Corn contains about 60-68% starch; barley contains about 55-65% starch; millet contains about 75-80% starch; wheat contains about 60-65% starch; and polished rice contains about 70-72% starch. Specifically contemplated starch substrates are cornstarch, wheat starch, and barley starch. The starch from a grain may be ground or whole and includes corn solids, such as kernels, bran and/or cobs. The starch may be highly refined raw starch or feedstock from starch refinery processes. Various starches also are commercially available. For example, cornstarch may be available from Cerestar, Sigma, and Katayama Chemical Industry Co. (Japan); wheat starch may be available from Sigma; sweet potato starch may be available from Wako Pure Chemical Industry Co. (Japan); and potato starch may be available from Nakaari Chemical Pharmaceutical Co. (Japan).

### 3.2. Milling

The starch substrate can be a crude starch from milled whole grain, which contains non-starch fractions, e.g., germ residues and fibers. Milling may comprise either wet milling or dry milling. In wet milling, whole grain can be soaked in water or dilute acid to separate the grain into its component parts, e.g., starch, protein, germ, oil, kernel fibers. Wet milling efficiently separates the germ and meal (*i.e*., starch granules and protein) and can be especially suitable for production of syrups. In dry milling, whole kernels are ground into a fine powder and processed without fractionating the grain into its component parts. Dry milled grain thus will comprise significant amounts of non-starch carbohydrate compounds, in addition to starch. Most ethanol comes from dry milling. Alternatively, the starch to be processed may be a highly refined starch quality, for example, at least about 90%, at least about 95%, at least about 97%, or at least about 99.5% pure.

### 3.3. Gelatinization and Liquefaction

In some embodiments of the invention, gelatinazation and/or liquefaction may be used. As used herein, the term "liquefaction" or "liquefy" means a process by which starch is converted to less viscous and soluble shorter chain dextrins. In some embodiments, this process involves gelatinization of starch simultaneously with or followed by the addition of alpha-amylases. Additional liquefaction-inducing enzymes, e.g., a phytase, optionally may be added. In some embodiments, gelatinization is not used. In other embodiments, a separate liquefaction step is not used. A separate liquefaction step may thus be used prior to the simultaneous saccharification and fermentation.

The starch substrate prepared as described above may be slurried with water. The starch slurry may contain starch as a weight percent of dry solids of about 10-55%, about 20-45%, about 30-45%, about 30-40%, or about 30-35%. In some embodiments, the starch slurry is at least about 5%, at least about 10%, at least about 15%, at least about 20%, at least about 25%, at least about 30%, at least about 35%, at least about 40%, at least about 45%, at least about 50%, or at least about 55%.

To optimize alpha-amylase stability and activity, the pH of the slurry may be adjusted to the optimal pH for the alpha-amylases. Alpha-amylases remaining in the slurry following liquefaction may be deactivated by lowering pH in a subsequent reaction step or by removing calcium from the slurry. The pH of the slurry should be adjusted to a neutral pH (e.g., pH 5.0 to 8.0 and any pH in between this range) when the glucoamylases of the invention are used.

The slurry of starch plus the alpha-amylases may be pumped continuously through a jet cooker, which may be steam heated from about 85°C to up to about 105°C. Gelatinization occurs very rapidly under these conditions, and the enzymatic activity, combined with the significant shear forces, begins the hydrolysis of the starch substrate. The residence time in the jet cooker can be very brief. The partly gelatinized starch may be passed into a series of holding tubes maintained at about 85-105°C and held for about 5 min. to complete the gelatinization process. These tanks may contain baffles to discourage back mixing. As used herein, the term "secondary liquefaction" refers the liquefaction step subsequent to primary liquefaction, when the slurry is allowed to cool to room temperature. This cooling step can be about 30 minutes to about 180 minutes, e.g., about 90 minutes to 120 minutes. Milled and liquefied grain is also known as mash.

### 3.4. Saccharification (is part of the SSF process of Claim 1)

The saccharification is carried out at a pH in the range of 6.0 to 7.5, 6.5 to 7.5, or 7.0 to 7.5, by using a glucoamylase as defined in Claim 1

At pH 6.0 or higher, the glucoamylase possesses at least 50%, about 51%, about 52%, about 53%, about 54%, or about 55% activity relative to its maximum activity at the optimum pH. For the pH range of 6.0 to 7.5, HgGA can have at least 53% activity relative to its maximum activity. Preferably, the glucoamylase has 67% maximal activity at pH 7.0.

The glucoamylase may be dosed at the range of about 0.2 to 2.0 GAU /g dss, about 0.5 to 1.5 GAU /g dss, or 1.0 to 1.5 GAU /g dss. The glucoamylase can also be used at a dose of about 0.25 to 1 GAU/ gds starch, for instance, 0.25 GAU/ gds starch, 0.5 GAU/ gds starch, 0.75 GAU/ gds starch, or 1 GAU/ gds starch. The saccharification may be performed at about 30 to about 60°C, or about 40 to about 60°C. In some embodiments, the saccharification occurs at pH 7.0 at 32°C. In other embodiments, the saccharification occurs at pH 6.5 at 58°C.

A full saccharification step may typically range 24 to 96 hours, 24 to 72 hours, or 24 to 48 hours. In some embodiments, saccharification occurs after about 2, 4, 6, 7.7, 8, 110, 14, 16, 18, 20, 22, 23.5, 24, 26, 28, 30, 31.5, 34, 36, 38, 40, 42, 44, 46, or 48 hours. In all embodiments, the saccharification step and fermentation step are combined and the process is referred to as simultaneous saccharification and fermentation (SSF).

It is understood that generally, as time elapses, the enzymes (glucoamylase with or without other enzymes, such as alpha-amylases or non-starch polysaccharide hydrolyzing enzyme) reduces the higher sugars to lower DP sugars (such as DP1). The sugar profile can be varied by using different parameters, such as, but not limited to, starting starch substrate, temperature, amount of glucoamylase, type of glucoamylase, and pH. For example, at 32 degrees Celsius and pH 7.0, the sugar or oligosaccharide distribution during the saccharification process can be between about 0.36% to about 96.50% DP1, about 3.59% to about 11.80% DP2, about 0.12% to about 7.75%, and/or about 2.26% to about 88.30% for higher sugars for HgGA. Thus, HgGA, the DP1 content can reach more than 90% after 24 hours. After 45 hours, the DP1 content can reach more than 96%, while the content of higher sugars can decreas to less than 3%.

At 58 degrees Celsius and pH 6.5, the sugar distribution during the saccharification process can be between about 60.66% to about 93.67% DP1, between about 1.49% to about 8.87% DP2, about 0.33% to about 1.93% DP3 and/or about 4.51% to about 28.17% for higher sugars for HgGA. Using HgGA, the DP1 content can reach more than 90% after 24 hours. After 48 hours, the DP1 content can reach more than 93%, while the content of higher sugars can decrease to less than 5%.

The sugar or oligosaccharide distribution during the saccharification process can be between about 93.15% to about 95.33% DP1, about 2.10% to about 3.94% DP2, about 0.53% to about 1.00% DP3, about 0.94% to about 3.76% DP4+ for HgGA.

At 58 degrees Celsius and pH 6.4, the sugar or oligosaccharide distribution during the saccharification process can be between about 93.79% to about 96.9% DP1, about 1.55% to about 3.02% DP2, about 0.2% to about 0.49% DP3 and about 0% to about 3.98% DP4+ for HgGA. In some cases, about 93% solubility and about 96.9% glucose yield can be achieved within 24 hours. Continuous saccharification can result in 99% solubility and about 96.8% glucose after about 48 hours.

At 58 degrees Celsius and pH 6.4, the sugar or oligosaccharide distribution during the saccharification process can be between about 75.08% to about 96.5% DP1, 1.57% to about 9.16% DP2, 0.67% to about 15.76% DP3+. In some cases, HgGA can maintain a significant amount of glucoamylase activity for about 52 hours at pH6.4 to yield continued production of DP1 products, DP2 products, and increase of percentage of soluble solids. Increased amounts of HgGA can result in increased rates of percentage solubilization and DP1 production.

### 3.5. Fermentation (is part of the SSF process of Claim 1)

As a technical background, fermentable sugars may be subject to batch or continuous fermentation conditions. A classical batch fermentation is a closed system, wherein the composition of the medium is set at the beginning of the fermentation and is not subject to artificial alterations during the fermentation. Thus, at the beginning of the fermentation the medium may be inoculated with the desired organism(s). In this method, fermentation can be permitted to occur without the addition of any components to the system. Typically, a batch fermentation qualifies as a "batch" with respect to the addition of the carbon source and attempts are often made at controlling factors such as pH and oxygen concentration. The metabolite and biomass compositions of the batch system change constantly up to the time the fermentation is stopped. Within batch cultures, cells progress through a static lag phase to a high growth log phase, and finally to a stationary phase where growth rate is diminished or halted. If untreated, cells in the stationary phase eventually die. In general, cells in log phase are responsible for the bulk of production of the end product.

A variation on the standard batch system is the "fed-batch fermentation" system. In this variation of a typical batch system, the substrate can be added in increments as the fermentation progresses. Fed-batch systems are particularly useful when catabolite repression is apt to inhibit the metabolism of the cells and where it is desirable to have limited amounts of substrate in the medium. Measurement of the actual substrate concentration in fed-batch systems may be difficult and is therefore estimated on the basis of the changes of measurable factors such as pH, dissolved oxygen and the partial pressure of waste gases such as CO₂. Both batch and fed-batch fermentations are common and well known in the art.

On the other hand, continuous fermentation is an open system where a defined fermentation medium can be added continuously to a bioreactor and an equal amount of conditioned medium can be removed simultaneously for processing. Continuous fermentation generally maintains the cultures at a constant high density where cells are primarily in log phase growth. Continuous fermentation allows for the modulation of one factor or any number of factors that affect cell growth and/or end product concentration. For example, a limiting nutrient such as the carbon source or nitrogen source can be maintained at a fixed rate while all other parameters are allowed to moderate. In other systems, a number of factors affecting growth can be altered continuously while the cell concentration, measured by media turbidity, may be kept constant. Continuous systems strive to maintain steady state growth conditions. Thus, cell loss due to medium being drawn off must be balanced against the cell growth rate in the fermentation. Methods of modulating nutrients and growth factors for continuous fermentation processes as well as techniques for maximizing the rate of product formation are well known in the art of industrial microbiology.

By use of appropriate fermenting microorganisms as known in the art, the fermentation end product may include without limitation alcohol, 1,3-propanediol, succinic acid, lactic acid, amino acids, proteins, functional oligosaccharides, and derivatives thereof. *See e*.*g*., WO 2008/086811 (methanol, ethanol, propanol, and butanol fermentation); WO 2003/066816, U.S. Patent Nos. 5,254,467 and 6,303,352 (1,3-propanediol fermentation); U.S. Patent Nos. RE 37,393, 6,265,190, and 6,596,521 (succinic acid fermentation); U.S. Patent No. 5,464,760, WO 2003/095659, Mercier et al., J. Chem. Tech. Biotechnol. 55: 111-121, Zhang and Cheryan, Biotechnol. Lett. 13: 733-738 (1991), Linko and Javanainen, Enzyme Microb. Technol. 19: 118-123 (1996), and Tsai and Moon, Appl. Biochem. Biotechnol. 70-72: 417-428 (1998) (lactic acid fermentation); U.S. Patent Nos. 7,320,882, 7,332,309, 7,666,634, and Zhang et al., Appl. Microbiol. Biotechnol. 77: 355-366 (2007) (fermentation of various amino acids). The above enumerated list are only examples and one skilled in the art will be aware of a number of fermenting microorganisms that may be appropriately used to obtain a desired end product.

### 3.6. Simultaneous saccharification and fermentation (SSF) (= the process of the present invention)

During SSF, the hydrolyzing enzymes are added along with the end product producer, commonly a microorganism. Enzymes release fermentable lower molecular weight sugars, *i.e.,* fermentable sugars DP1-3, from the starch substrate, while the microorganism simultaneously uses the fermentable sugars for growth and production of the end product. Typically, fermentation conditions are selected that provide an optimal pH and temperature for promoting the best growth kinetics of the producer host cell strain and catalytic conditions for the enzymes produced by the culture. *See e.g.,* Doran et al., Biotechnol. Progress 9: 533-538 (1993). Table 1 presents exemplary fermentation microorganism and their optimal pH for fermentation. Because the glucoamylases disclosed herein, and defined in Claim 1, possess significant activity at a neutral pH and an elevated temperature, they would be useful in the SSF for those microorganisms having an optimal fermenting pH in the range of 6.0 to 7.5.

**Table 1. Exemplary fermentation organisms and their optimal pH.**

| **End products** | **Fermentation Organisms** | **Optimal pH of the fermentation** |
|---|---|---|
| **Lysine and salts thereof** | *Corynebacterium glutamicum* | 6.8-7.0 |
| | *Bacillus lacterosprous* | 7.0-7.2 |
| | *Methylophilotrophus* | 7 |
| **Lactic Acid** | *Lactobacillus amylophilus* | 6.0-6.5 |
| | *Bacillus coagulans* | 6.4-6.6 |
| | *Bacillus thermoamylovorans* | 5.0-6.5 |
| | *Bacillus smithii* | 5.0-6.5 |
| | *Geobacillus stearothermophilus* | 5.0-6.5 |
| **Monosodium Glutamate (MSG)** | *Corynebacterium pekinense* | 7 |
| | *Corynebacterium crenatum* | 7 |
| | *Brevibacterium tianjinese* | 7 |
| | *Corynebacterium glutamicum* HU7251 | 7.0-7.2 |
| | *Arthrobacter sp* | 7 |
| **Succinic acid** | *Escherichia coli* | 6.0-7.5 |
| **1,3-Propanediol** | *Escherichia coli* | 6.5-7.5 |
| **2-Keto-gulonic acid** | *Escherichia coli* | 5.0-6.0 |

In further embodiments, by use of appropriate fermenting microorganisms as known in the art to produce the desired end product, those of skill in the art are well capable of adjusting the SSF conditions, *e*.*g*., temperature, nutrient composition, light conditions, oxygen availability, etc.

### 4. Methods used in the Examples

The following materials, assays, and methods are used in the examples provided below:

### HPLC method to measure saccharide composition

The composition of the reaction products of oligosaccharides was measured by a HPLC system (Beckman System Gold 32 Karat Fullerton, CA). The system, maintained at 50°C, was equipped with a Rezex 8 u8% H Monosaccharides column and a refractive index (RI) detector (ERC-7515A, Anspec Company, Inc.). Diluted sulfuric acid (0.01 N) was applied as the mobile phase at a flow rate of 0.6 ml/min. 20 µl of 4.0% solution of the reaction mixture was injected onto the column. The column separates saccharides based on their molecular weights. The distribution of saccharides and the amount of each saccharide were determined from previously run standards.

### Determination of glucoamylase activity units (GAU)

Glucoamylase activity units (GAU) were determined based on the activity of a glucoamylase enzyme to catalyze the hydrolysis of *p*-nitrophenyl-alpha-D-glucopyranoside (PNPG) to glucose and p-nitrophenol. At an alkaline pH, *p*-nitrophenol forms a yellow color that is measured spectrophotometrically at 405 nm. The amount of p-nitrophenol released correlates with the glucoamylase activity.

### Protein concentration determination

The protein concentration in a sample was determined using the Bradford QuickStart™ Dye Reagent (Bio-Rad, California, USA). For example, a 10 µL sample of the enzyme was combined with 200 µL Bradford QuickStart™ Dye Reagent. After thorough mixing, the reaction mixture was incubated for at least 10 minutes at room temperature. Air bubbles were removed and the optical density (OD) was measured at 595 nm. The protein concentration was then calculated using a standard curve generated from known amounts of bovine serum albumin.

### Purification of HgGA for characterization studies

The material concentrated by ultrafiltration (UFC) was desalted / buffer-exchanged using a BioRad DP-10 desalting column and 25 mM Tris pH 8.0. 100 mg of total protein was applied to a Pharmacia Hi Prep 16/10 S Sepharose FF column, which was equilibrated with the above buffer at 5 ml/min. Glucoamylase was eluted with a 4-column volume (CV) gradient buffer containing 0-200 mM NaCl. Multiple runs were performed and the purest fractions, as determined via SDS-PAGE/coomassie blue staining analysis, were pooled and concentrated using VivaSpin 10K MWCO 25 ml spin tubes. The final material was passed over a Novagen HisBind 900 chromatography cartridge that had been washed with 250 mM EDTA and rinsed with above buffer. 2 ml of final material was obtained, having a protein concentration of 103.6 mg/ml, and a glucoamylase activity of 166.1 GAU/ml (determined by a PNPG based assay). Specific activities were determined using a standardized method using *p*-nitrophenyl-alpha-D-glucopyranoside (PNPG) as a substrate and reported in GAU units.

### Determination of glucose concentration

Glucose concentration in a saccharification reaction mixture was determined with the ABTS assay. Samples or glucose standards in 5 µL were placed in wells of a 96-well microtiter plate (MTP). Reactions were initiated with the addition of 95 µL of the reactant containing 2.74 mg/ml 2,2'-Azino-bis(3-ethylbenzothiazoline-6-sulfonic acid) diammonium salt (ABTS) (Sigma P1888), 0.1 U/ml horseradish peroxidase type VI (Sigma P8375), and 1 U/ml glucose oxidase (Sigma G7141). OD_{405 nm} was immediately monitored at a 9-second interval for 300 seconds using a Spectramax plate reader. Because the rate of OD₄₀₅ₙₘ increase is proportional to the glucose concentration, the sample's glucose concentration was determined by comparing with the glucose standard, and was reported as mg/ml.

### Lactic acid fermentation conditions

Strains of *Lactobacillus rhamnosus* and *Bacillus coagulans* were obtained from China General Microbiological Culture Collection Center.

Seed media (MRS 006): Casein 10.0 g, Beef extract 10.0 g, Yeast extract 5.0 g, Glucose 5.0 g, Sodium acetate 5.0 g, diammonium citrate 2.0 g, Tween® 80 1.0 g, K₂HPO₄ 2.0 g, MgSO₄·7H₂O 0.2 g, MnSO₄.H₂O 0.05 g, Distilled water 1.0 L, pH 6.8; additional 20 g agar were added for inoculum media.

Fermentation medium: corn steep liquor 40 g, casein 10.0 g, Beef extract 10.0 g, Yeast extract 10.0 g, cassava starch 150 g, Tween® 80 1.5 g, MnSO₄·H₂O 0.3 g, Calcium carbonate 20 g, Distilled water 1.0 L, pH 6.5. Starch (cassava starch or cornstarch) or glucose was added based on different test conditions.

Starch (cassava starch, cornstarch, or wheat starch), glucose, agar, corn steep liquor, casein, Beef extract, Yeast extract, Glucose, Sodium acetate, diammonium hydrogen citrate, Tween® 80, K₂HPO₄, MgSO₄·7H₂O, MnSO₄·H₂O, CaCO₃, and Agar powder were all of analytic grade and procured locally.

The inoculum of *Lactobacillus rhamnosus* was transferred to each 100 mL seed culture and cultivated at 37°C, 200 rpm, for 12 to 24 hrs, until OD₆₀₀ reached about 0.5. 10-20 mL seed culture was added to each fermentor with 1 L fermentation medium.

### EXAMPLES

### Example 1: Comparison of the pH and activity profiles of various glucoamylases at 32°C

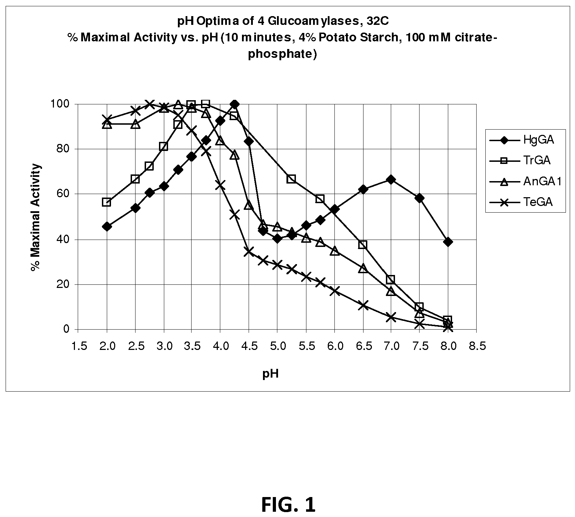

The pH and activity profiles of glucoamylases (GAs) from *Humicola grisea* (HgGA), *Trichoderma reesei* (TrGA), *Aspergillus niger* (AnGA) and *Talaromyces emersonii* (TeGA) were determined at 32°C. As the substrate, 8% potato starch (Sigma Cat. No. S2630) was solubilized by heating. A series of citrate/phosphate buffers at 0.25 or 0.5 pH increments, ranging from pH 2.0 to 8.0, were prepared. Purified enzymes were diluted to 0.1 or 0.02 GAU/ml in water (TeGA was dosed at 0.2 GAU/ml). HgGA, TrGA, AnGA, and TeGA were dosed at 0.0125, 0.0076, 0.0109, and 0.0055 mg/ml, respectively. 10 µL buffer of various pH was placed in 0.2 ml PCR tube strips (AB Gene, Cat. No. AB-0451, 800-445-2812) with 15 µL of diluted enzyme. The reactions were initiated by the addition of 25 µL soluble potato starch. The reactions were incubated on a PCR type thermocycler heating block for exactly ten minutes, then terminated by the addition of 10 µL 0.5 M NaOH. The glucose released in the reaction was determined using the ABTS assay, and the glucoamylase activities were determined. The pH and activity profiles are presented in Table and FIG. 1 as the percentage of the maximum activity for each glucoamylase.

**Table 2. pH profiles of HgGA, TrGA, AnGA, and TeGA at 32°C. The values represent % of the maximum activity for each enzyme.**

| **pH** | **HgGA** | **TrGA** | **AnGA** | **TeGA** |
|---|---|---|---|---|
| 2.00 | 45 | 56 | 91 | 93 |
| 2.50 | 54 | 67 | 91 | 97 |
| 2.75 | 60 | 72 | | 100 |
| 3.00 | 63 | 81 | 98 | 98 |
| 3.25 | 71 | 91 | 100 | 95 |
| 3.50 | 77 | 99 | 99 | 88 |
| 3.75 | 84 | 100 | 96 | 79 |
| 4.00 | 93 | | 84 | 64 |
| 4.25 | 100 | 95 | 78 | 51 |
| 4.50 | 84 | | 55 | 34 |
| 4.75 | 44 | | 46 | 30 |
| 5.00 | 40 | | 45 | 29 |
| 5.25 | 42 | 66 | 43 | 27 |
| 5.50 | 46 | | 41 | 23 |
| 5.75 | 48 | 58 | 39 | 21 |
| 6.00 | 53 | 51 | 35 | 17 |
| 6.50 | 62 | 38 | 27 | 11 |
| 7.00 | 67 | 22 | 17 | 5 |
| 7.50 | 58 | 10 | 7 | 2 |
| 8.00 | 39 | 4 | 3 | 1 |

As shown in Table 2 and FIG. 1, both TeGA and AnGA exhibited significantly reduced activity in the pH range of 6.0 to 8.0. At a pH 5.0 or above, TeGA retained no more than 29% activity relative to its maximum activity. At a pH 6.0 or above, TeGA retained no more than 17% activity relative to its maximum activity. Similarly, at a pH 6.0 of 6.0 or above, AnGA displayed no more than 35% activity relative to its maximum activity. In the pH range of 6.0 to 7.5, HgGA retained at least 53% activity relative to its maximum activity. At pH 6.0, TrGA also displayed at least 50% activity relative to its maximum activity. The above observation suggests that both HgGA and TrGA may be suitable for producing fermentable sugars at a neutral pH range (as described herein for neutral pH glucoamylases) under fermentation conditions.

### Example 2: Comparison of hydrolysis of solubilized starch at 32°C, pH 7.0

The ability of various glucoamylases to hydrolyze solubilized starch substrate (liquefact) at a neutral pH was compared. Corn starch was liquefied by following a conventional high-temperature jet cooking process using CLEARFLOW™ AA to a liquefact of DE 12-15. Saccharification of the liquefact (25% DS) was carried out using TrGA, HgGA, and AnGA at 1.0 GAU/g ds at 32°C, pH 7.0. Samples were withdrawn at different time intervals during the saccharification and subject to HPLC analysis. The composition of the oligosaccharides is presented in Table 3.

**Table 3. Composition of oligosaccharides in saccharification.**

| **GA** | **Time (hr)** | **% Sugars, pH 7.0, 32°C** | | | |
|---|---|---|---|---|---|
| | | **DP1** | **DP2** | **DP3** | **Higher Sugars** |
| **HgGA** | 0 | 0.36 | 3.59 | 7.75 | 88.30 |
| | 2 | 51.10 | 10.20 | 6.87 | 31.85 |
| | 5.25 | 64.90 | 11.80 | 0.13 | 23.13 |
| | 21.25 | 89.30 | 1.10 | 0.30 | 9.34 |
| | 25.25 | 91.20 | 0.98 | 0.23 | 7.61 |
| | 29.25 | 92.60 | 0.90 | 0.31 | 6.12 |
| | 45.25 | 96.50 | 1.15 | 0.12 | 2.26 |
| | | | | | |
| **TrGA** | 0 | 0.36 | 3.59 | 7.75 | 88.30 |
| | 2 | 38.06 | 7.49 | 9.10 | 45.35 |
| | 5.25 | 47.17 | 9.92 | 6.13 | 36.78 |
| | 21.25 | 69.43 | 8.33 | 0.17 | 22.07 |
| | 25.25 | 71.69 | 7.14 | 0.17 | 21.01 |
| | 29.25 | 73.57 | 6.16 | 0.18 | 20.09 |
| | 45.25 | 79.19 | 3.45 | 0.20 | 17.15 |
| | | | | | |
| **AnGA** | 0 | 0.36 | 3.59 | 7.75 | 88.30 |
| | 2 | 14.12 | 4.57 | 8.88 | 72.43 |
| | 5.25 | 28.38 | 8.01 | 10.30 | 53.31 |
| | 21.25 | 58.97 | 11.49 | 0.28 | 29.26 |
| | 25.25 | 60.94 | 10.53 | 0.28 | 28.25 |
| | 29.25 | 62.82 | 9.54 | 0.23 | 27.41 |
| | 45.25 | 74.14 | 4.08 | 0.24 | 21.54 |

Using HgGA, the DP1 content reached more than 90% after 24 hrs. After 45 hours, the DP1 content reached more than 96%, while the content of higher sugars decreased to less than 3%. Using TrGA, more than 70% DP1 was obtained after 24 hours. After 45 hours, the DP1 content reaches about 80%, while the content of higher sugars dropped to less than 20%. For AnGA, less than 75% of DP1 was obtained after 45 hours, while higher sugars remained more than 20%. The data in Table 3 indicate that both HgGA and TrGA are more effective than AnGA to hydrolyze solubilized starch to glucose, at a neutral pH.

### Example 3: Comparison of hydrolysis of liquefied starch at 58°C, pH 6.5

Corn starch liquefact (∼9.1DE) obtained by SPEZYME^{®} FRED (Danisco US Inc., Genencor Division) treatment was adjusted to pH 6.5 with NaOH and equilibrated at a 58°C water bath. AnGA (OPTIDEX™ L-400, Danisco US Inc., Genencor Division), TrGA, and HgGA were added at 0.5 GAU/g ds to each flask containing corn starch liquefact. Saccharification was carried out up to 48 hours with periodical sampling for HPLC analysis. 0.5 mL enzyme-deactivated sample was diluted with 4.5 ml of RO water. The diluted sample was then filtered through 0.45 µm Whatman filters and subject to HPLC analysis. The HPLC analysis was conducted as described in Methods used in the Examples. The composition of the oligosaccharides is presented in Table 4.

**Table 4. Composition of oligosaccharides in saccharification.**

| | **Hour** | **Percent Sugar Composition** | | | |
|---|---|---|---|---|---|
| | | %DP1 | %DP2 | %DP3 | %HS |
| **Liquefact** | 0 | 0.49 | 3.02 | 5.52 | 90.98 |
| | | | | | |
| **HgGA** | 2 | 60.66 | 8.87 | 1.93 | 28.17 |
| | 4 | 69.92 | 7.43 | 0.69 | 21.75 |
| | 6 | 75.96 | 5.80 | 0.38 | 17.85 |
| | 7.7 | 77.56 | 5.15 | 0.47 | 16.35 |
| | 14 | 84.31 | 2.96 | 0.42 | 11.57 |
| | 23.5 | 88.70 | 2.20 | 0.43 | 8.67 |
| | 31.5 | 90.01 | 1.87 | 0.40 | 6.90 |
| | 48 | 93.67 | 1.49 | 0.33 | 4.51 |
| | | | | | |
| **TrGA** | 2 | 37.08 | 10.19 | 5.06 | 47.47 |
| | 4 | 49.25 | 12.12 | 2.12 | 36.42 |
| | 6 | 55.30 | 12.16 | 1.09 | 31.10 |
| | 7.7 | 58.06 | 11.74 | 0.76 | 29.12 |
| | 14 | 63.83 | 9.96 | 0.46 | 25.28 |
| | 23.5 | 68.52 | 8.18 | 0.53 | 22.77 |
| | 31.5 | 70.35 | 7.24 | 0.54 | 21.32 |
| | 48 | 75.25 | 5.48 | 0.50 | 18.37 |
| | | | | | |
| **AnGA** | 2 | 41.33 | 11.83 | 4.40 | 42.20 |
| | 4 | 50.08 | 12.95 | 1.60 | 35.04 |
| | 6 | 53.32 | 12.70 | 0.83 | 33.16 |
| | 7.7 | 54.80 | 12.41 | 0.62 | 31.91 |
| | 14 | 58.85 | 11.20 | 0.40 | 29.15 |
| | 23.5 | 61.70 | 10.44 | 0.46 | 27.41 |
| | 31.5 | 62.34 | 10.11 | 0.50 | 26.58 |
| | 48 | 64.23 | 9.83 | 0.59 | 25.01 |

Using HgGA, the DP1 content reached more than 90% after 24 hrs. After 48 hours, the DP1 content reached more than 93%, while the content of higher sugars decreased to less than 5%. Using TrGA, more than 70% DP1 was obtained after 24 hours. After 45 hours, the DP1 content reaches about 75%, while the content of higher sugars dropped to about 18%. For AnGA, less than 65% of DP1 was obtained after 45 hours, while higher sugars remained more than 25%. The data in Table 4 indicate that both HgGA and TrGA are more effective than AnGA, at a neutral pH and 58°C, to hydrolyze solubilized starch to glucose. This observation is consistent with data presented in Table 3, where saccharification was performed at 32°C.

### Example 4: Comparison of high sugars (DP4+) reduction at 58°C, pH 6.5

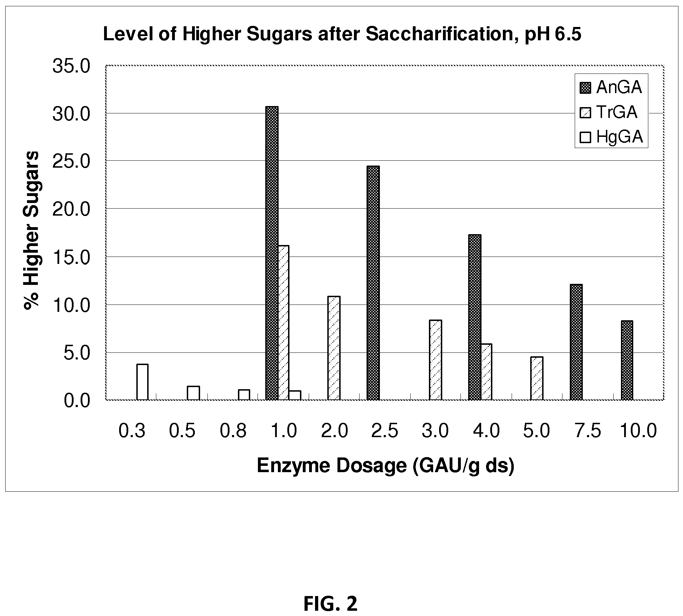

Various concentrations of AnGA, TrGA, and HgGA were used to saccharify a starch substrate at 58°C, pH 6.5, and the reduction of high sugars (DP4+) was compared. The starch substrate was a 25% cornstarch liquefact, which was liquefied by SPEZYME^{®} FRED (Danisco US Inc., Genencor Division). Glucoamylases were added as shown in Table 5, from 0.25 GAU/gds to 10.0 GAU/gds. The saccharification reaction was conducted at 58°C, pH 6.5. Samples were withdrawn at various time points and the sugar composition was determined by HPLC analysis. The composition of the oligosaccharides is presented in Table 5 and FIG. 2.

**Table 5. Composition of oligosaccharides in saccharification.**

| **Glucoamylase** | **GAU/gds starch** | **Percent Sugar Composition at 48 hr** | | | |
|---|---|---|---|---|---|
| | | **DP1** | **DP2** | **DP3** | **DP4+** |
| **AnGA** | 1 | 64.25 | 5.10 | 0.00 | 30.65 |
| | 2.5 | 73.36 | 1.74 | 0.41 | 24.49 |
| | 5 | 81.26 | 1.05 | 0.46 | 17.22 |
| | 7.5 | 85.53 | 1.48 | 0.44 | 12.13 |
| | 10 | 89.32 | 2.03 | 0.42 | 8.22 |
| | | | | | |
| **TrGA** | 1 | 81.10 | 2.28 | 0.49 | 16.13 |
| | 2 | 86.65 | 1.99 | 0.49 | 10.87 |
| | 3 | 90.36 | 2.86 | 0.49 | 8.30 |
| | 4 | 90.48 | 3.17 | 0.52 | 5.83 |
| | 5 | 90.95 | 3.96 | 0.61 | 4.48 |
| | | | | | |
| **HgGA** | 0.25 | 93.15 | 2.10 | 1.00 | 3.76 |
| | 0.5 | 95.33 | 2.58 | 0.64 | 1.45 |
| | 0.75 | 95.08 | 3.36 | 0.53 | 1.02 |
| | 1 | 94.57 | 3.94 | 0.56 | 0.94 |

The results presented in Table 5 and FIG. 2 indicated that AnGA resulted in more than 8% of higher sugars (DP4+), at 58°C, pH 6.5, even at a high dosage of glucoamylase, 10.0 GAU/gds. In contrast, lower than 5% of higher sugars (DP4+) was observed for 5 GAU/gds TrGA. HgGA resulted in the lowest levels of higher sugars (DP4+). For example, at 0.5 GAU/gds HgGA, the saccharification mixture contained less than 1.5% of higher sugars (DP4+), which is comparable to the resulted obtained under the current industrial high glucose processing conditions (pH 4.5, 60°C) using AnGA.

### Example 5: Continuous production of glucose from granular Cassava starch by HgGA at a neutral pH

The capability of HgGA to convert granular unmodified cassava starch to glucose and short chain glucose polymers at a neutral pH was further characterized. A 27 % dry substance aqueous slurry of cassava starch was first adjusted to pH 6.4 with sodium carbonate. SPEZYME™ Alpha (Danisco US Inc., Genencor Division) was added at 2 AAU/g ds, and HgGA was added at 1 GAU/g ds. The reaction was carried out for 48 hours at 58 °C with continuous stirring. At selected time intervals, samples of the slurry were removed. The removed sample was added to a 2.5 ml micro-centrifuge tube and centrifuged for 4 minutes at 13,000 rpm. Refractive index (RI) of the supernatant was determined at 30°C. The remaining supernatant was filtered through a 13 mm syringe filter with a 0.45 µm GHP membrane into a 2.5 ml micro-centrifuge tube and boiled for 10 minutes to terminate the amylase activity. 0.5 mL enzyme-deactivated sample was diluted with 4.5 ml of RO water. The diluted sample was then filtered through 0.45 µm Whatman filters and subject to HPLC analysis. The HPLC analysis was conducted as described in Methods used in the Examples.

The total dry substance was determined by taking about 1 ml of the starch slurry into a 2.5 ml spin tube, adding 1 drop of SPEZYME^{®} FRED (Danisco US Inc., Genencor Division) from a micro dispo-pipette, and boiling 10 minutes. Refractive index at 30°C was determined. The dry substance of the supernatant and the whole sample (total) was determined using appropriate DE tables. The CRA 95 DE Table was used for the supernatant and corrected for consumption of water of hydrolysis. % soluble was calculated as: 100 x (the dry substance of the supernatant) / (the total dry substance). The composition of the oligosaccharides is presented in Table 6.

**Table 6 Saccharide distribution for HgGA-mediated saccharification of cassava granular starch.**

| **hrs** | **Saccharide Distribution** | | | | **Soluble %** |
|---|---|---|---|---|---|
| | **DP1** | **DP2** | **DP3** | **DP4+** | |
| 2.50 | 93.799 | 1.726 | 0.499 | 3.976 | 56.20 |
| 7.50 | 96.166 | 1.551 | 0.480 | 1.802 | 78.80 |
| 12.00 | 96.731 | 1.639 | 0.411 | 1.220 | 85.10 |
| 23.50 | 96.928 | 2.204 | 0.326 | 0.541 | 92.80 |
| 48.00 | 96.772 | 3.023 | 0.205 | 0.000 | 99.00 |

As shown in Table 6, the reaction achieved about 93% solubility and yielded about 96.9% glucose within 24 hours. Continuation of saccharification resulted in 99% solubility and about 96.8% glucose after 48 hours.

### Example 6: Continuous production of glucose from granular cornstarch by HgGA at a neutral pH

Corn granular starch was used to characterize HgGA. The experiments were carried out using 32% ds corn granular starch. Water (64.44 g) and starch (35.56 g; at 90% ds) were mixed and the pH of the slurry was increased to 6.4. The starch slurry was placed in a water bath maintained at 58°C and enzymes were added. The enzymes included SPEZYME™ Alpha (Danisco US Inc., Genencor Division) and HgGA. The starch slurry was maintained at 58°C for 48 hrs and samples were drawn at 3, 6, 10, 24, 32, and 52 hrs to analyze the % soluble and saccharide profile. The results are presented in Table 7.

**Table 7. Saccharide distribution for HgGA-mediated saccharification of corn granular starch**

| **HgGA (GAU/q ds)** | **Alpha-amylase (AAU/g ds)** | **hour** | **% Soluble** | **DP1** | **DP2** | **DP3+** |
|---|---|---|---|---|---|---|
| 1 | 2 | 3 | 56.82 | 94.74 | 1.57 | 3.69 |
| | | 6 | 69.45 | 95.52 | 1.76 | 2.61 |
| | | 10 | 75.96 | 96.50 | 1.79 | 1.43 |
| | | 24 | 91.50 | 95.72 | 2.79 | 0.93 |
| | | 32 | 92.71 | 95.50 | 3.08 | 0.86 |
| | | 52 | 99.66 | 93.94 | 4.42 | 0.67 |
| | | | | | | |
| 0.75 | 2 | 3 | 53.35 | 92.74 | 2.00 | 5.25 |
| | | 6 | 65.87 | 94.69 | 1.77 | 3.43 |
| | | 10 | 73.11 | 95.80 | 1.73 | 2.12 |
| | | 24 | 89.09 | 95.70 | 2.53 | 1.59 |
| | | 32 | 91.01 | 95.75 | 2.64 | 1.01 |
| | | 52 | 98.65 | 95.44 | 3.44 | 1.12 |
| | | | | | | |
| 0.5 | 2 | 3 | 49.06 | 88.36 | 3.36 | 8.29 |
| | | 6 | 61.98 | 92.48 | 2.18 | 5.35 |
| | | 10 | 68.18 | 94.08 | 1.90 | 3.67 |
| | | 24 | 84.14 | 95.56 | 2.03 | 2.23 |
| | | 32 | 87.90 | 95.49 | 2.25 | 2.11 |
| | | 52 | 95.17 | 95.30 | 2.81 | 1.12 |
| | | | | | | |
| 0.25 | 2 | 3 | 44.01 | 75.08 | 9.16 | 15.76 |
| | | 6 | 53.92 | 84.31 | 5.25 | 10.45 |
| | | 10 | 60.97 | 88.25 | 3.72 | 7.81 |
| | | 24 | 76.63 | 93.11 | 2.25 | 4.48 |
| | | 32 | 80.00 | 93.66 | 2.17 | 4.05 |
| | | 52 | 88.37 | 94.55 | 2.31 | 2.89 |

As shown in Table 7, HgGA maintains a significant amount of glucoamylase activity for 52 hrs at pH 6.4, evidenced by the continued production of DP1 and DP2, as well as the continued increase of % soluble solids. The data also suggest that the rates of DP1 production and % solubilization of granular starch depend on the amount of HgGA. An increased amount of HgGA resulted in increased rates of % solubilization and DP1 production.

### Example 7: Characterization of granular starch hydrolysis by HgGA and SPEZYME™ Alpha at a neutral pH by scanning electron microscopy

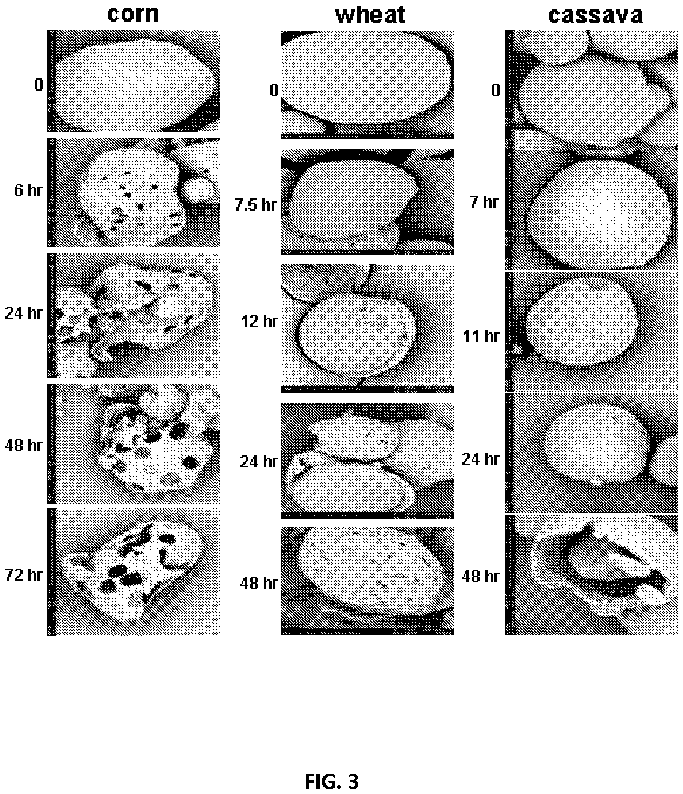

Granular starch from corn, wheat, and cassava was treated with HgGA and SPEZYME™ Alpha. A 28 % dry substance aqueous slurry of granular starch was first adjusted to pH 6.4 with sodium carbonate. SPEZYME™ Alpha (Danisco US Inc., Genencor Division) was added at 2 AAU/g ds, and HgGA was added at 1 GAU/g ds. Treatment was carried out at 58 °C with continuous stirring. Samples of the slurry were removed at various time points and subject to scanning electron microscopy (SEM). Slurry samples were laid on SEM sample stubs using double-sided carbon tape. Excess sample was removed by gently dusting the mounted sample with compressed air. Mounted samples were sputter coated with gold (15 nm) for 2 min at 25 mV, using an Emitech K550 Sputter Coater (Squorum Technologies). The scanning electron micrographs are presented in FIG. 3. Before treatment, starch surface was smooth and homogenous. Upon HgGA and SPEZYME™ Alpha treatment, the surface morphology of the granules changed over time. The enzyme blend first created small dimples (0.2-0.5 µm in diameter) on the surface of the starch granules. Quantity and size of the dimples increased over time. At a late stage of the treatment, for example, 48 hours for cassava granular starch, empty shells were spotted. Micrographs of empty shells indicated a complete digestion of the interior of the granule. The mechanism of enzymatic action appears to be starch granule surface peeling. Once the surface has been weakened by external peeling, the amylases penetrate and hydrolyze the interior of the granule (*i*.*e*., amylolysis) leaving hollowed out shells.

### Example 8: Lactic acid fermentation using various glucoamylases with the SSF process

Glucoamylases from various sources were tested for their use in lactic acid fermentation under a neutral pH. Lactic acid fermentation was carried out using SSF process. The substrate was 15% ds of cassava starch. Liquefaction was carried out at 85°C for 90 min with the alpha-amylase GC 358 (Danisco US Inc., Genencor Division) dosed at 0.25 kg/ton. For saccharification and fermentation, 0.3 GAU/g HgGA, 0.3 GAU/g OPTIDEX™ L-400 *Aspergillus niger* glucoamylase (AnGA) (Danisco US Inc., Genencor Division), and 0.3 GAU/g of TrGA was used. The SSF were carried out at pH of 6.5 at 40°C, using inoculum of *Lactobacillus rhamnosus.* Samples were withdrawn at various time points for HPLC analysis, and the results are presented in Table 8.

**Table 8. Lactic acid production during SSF**

| | **time(h)** | **DP >3 w/v%** | **DP-3 w/v%** | **DP-2 w/v%** | **Glucose w/v%** | **Fructose %w/v** | **succinic acid %w/v** | **lactic acid mg/mL** | **glycerol w/v%** | **acetic acid w/v%** | **ethanol w/v%** | **lactic acid g** |
|---|---|---|---|---|---|---|---|---|---|---|---|---|
| **AnGA** | 4 | 3.80 | 0.67 | 0.00 | 6.40 | 0.05 | 0.11 | 9.71 | 0.00 | 0.08 | 0.08 | 10.68 |
| | 21 | 3.36 | 0.00 | 0.00 | 1.23 | 0.00 | 0.08 | 78.62 | 0.00 | 0.00 | 0.16 | 95.13 |
| | 45 | 2.83 | 0.00 | 0.17 | 0.19 | 0.00 | 0.09 | 106.41 | 0.00 | 0.00 | 0.18 | 130.89 |
| | | | | | | | | | | | | |
| **TrGA** | 4 | 0.31 | 0.00 | 0.29 | 0.99 | 0.00 | 0.00 | 9.10 | 0.00 | 0.03 | 0.00 | 10.01 |
| | 21 | 0.15 | 0.03 | 0.04 | 0.32 | 0.00 | 0.00 | 88.73 | 0.00 | 0.04 | 0.04 | 107.36 |
| | 45 | 0.06 | 0.02 | 0.01 | 0.07 | 0.01 | 0.00 | 116.97 | 0.00 | 0.03 | 0.06 | 143.88 |
| | | | | | | | | | | | | |
| **HgGA** | 4 | 3.75 | 0.65 | 0.00 | 6.94 | 0.00 | 0.11 | 9.64 | 0.00 | 0.08 | 0.06 | 10.60 |
| | 21 | 2.75 | 0.00 | 0.00 | 1.19 | 0.00 | 0.09 | 79.92 | 0.00 | 0.00 | 0.18 | 96.70 |
| | 45 | 1.51 | 0.43 | 0.56 | 0.16 | 0.00 | 0.09 | 108.53 | 0.00 | 0.00 | 0.23 | 133.49 |

Data presented in Table 8 indicates that when same amount of glucoamylases were used, both HgGA and TrGA resulted in more lactic acid production than AnGA at a neutral pH.

### Example 9: Effect of alpha-amylase on lactic acid fermentation in no-cook process

Various alpha-amylases were combined with TrGA to produce lactic acid from cornstarch through a no-cook process. The lactic acid fermentation was performed with 2 GAU/g or 1 GAU/g TrGA glucoamylase, combined with various alpha-amylases, fungal alpha-amylase GC 626, themostable bacterial alpha amylase SPEZYME® XTRA, and bacterial amylase AmyE (all from Danisco US Inc., Genencor Division). Each alpha-amylase was dosed at 1 kg/MT. Raw cornstarch was used as the substrate at 15% DS. Fermentation was carried out at 40°C for 45 hrs using inoculum of *Lactobacillus rhamnosus.* Samples were withdrawn at various time points for HPLC analysis, and the results are presented in Table 9.

**Table 9. Lactic acid production during SSF**

| | **time(h)** | **DP >3 w/v%** | **DP-3 w/v%** | **DP-2 w/v%** | **Glucose w/v%** | **Fructose %w/v** | **succinic acid %w/v** | **lactic acid mg/mL** | **glycerol w/v%** | **acetic acid w/v%** | **ethanol w/v%** | **lactic acid 9** |
|---|---|---|---|---|---|---|---|---|---|---|---|---|
| 1 **kg/MT GC626** | 3.5 | 0.41 | 0.00 | 0.00 | 2.01 | 0.00 | 0.01 | 6.95 | 0.00 | 0.00 | 0.00 | 7.65 |
| | 21 | 0.30 | 0.00 | 0.04 | 1.19 | 0.01 | 0.04 | 33.43 | 0.00 | 0.21 | 2.11 | 38.44 |
| | 28 | 0.38 | 0.00 | 0.00 | 0.00 | 0.06 | 0.09 | 39.00 | 0.00 | 0.25 | 2.48 | 45.24 |
| | 45.5 | 0.42 | 0.00 | 0.00 | 0.00 | 0.08 | 0.18 | 43.67 | 0.00 | 0.31 | 2.59 | 51.53 |
| | | | | | | | | | | | | |
| **1 kg/MT SPEZYME® XTRA** | 3.5 | 9.31 | 0.00 | 1.25 | 7.64 | 0.35 | 0.21 | 9.24 | 0.00 | 0.38 | 0.29 | 10.17 |
| | 21 | 8.50 | 0.00 | 0.00 | 0.04 | 0.25 | 0.30 | 39.61 | 0.07 | 3.00 | 1.77 | 45.94 |
| | 28 | 11.91 | 0.00 | 0.06 | 0.05 | 0.15 | 0.30 | 47.91 | 0.10 | 3.92 | 5.40 | 56.54 |
| | 45.5 | 12.33 | 0.00 | 0.05 | 0.03 | 0.13 | 0.26 | 49.34 | 0.00 | 3.61 | 6.76 | 59.71 |
| | | | | | | | | | | | | |
| **1 kb/MT AmyE** | 3.5 | 0.30 | 0.00 | 0.17 | 0.55 | 0.07 | 0.03 | 9.13 | 0.00 | 0.02 | 0.00 | |
| | 21 | 0.29 | 0.07 | 0.03 | 0.01 | 0.03 | 0.04 | 44.05 | 0.00 | 0.15 | 0.10 | 10.04 |
| | 28 | 0.29 | 0.12 | 0.02 | 0.00 | 0.02 | 0.03 | 55.46 | 0.00 | 0.15 | 0.10 | 51.54 |
| | 45.5 | 0.37 | 0.13 | 0.02 | 0.03 | 0.01 | 0.04 | 72.91 | 0.00 | 0.28 | 0.21 | 66.00 |

Data presented in Table 9 indicate that AmyE combined with TrGA resulted in the highest lactic acid yield, while fungal alpha-amylase GC 626 resulted in the lowest lactic acid yield.

Furthermore, the lactic acid fermentation was further performed using 1 kg/t AmyE with 1 GAU/g of HgGA, AnGA, or TrGA with the no-cook process. The fermentation was performed at pH 6.5, 40°C, with 15% DS of cornstarch. Samples were withdrawn at various time points for HPLC analysis, and the results are presented in Table 10.

**Table 10. Lactic acid production during SSF**

| | **time(h)** | **DP >3 w/v%** | **DP-3 w/v%** | **DP-2 w/v%** | **Glucose w/v%** | **Fructose %w/v** | **succinic acid %w/v** | **lactic acid mg/mL** | **glycerol w/v%** | **acetic acid w/v%** | **ethanol w/v%** |
|---|---|---|---|---|---|---|---|---|---|---|---|
| **HgGA** | 5 | 0.32 | 0.00 | 0.04 | 1.06 | 0.11 | 0.05 | 17.96 | 0.00 | 0.04 | 0.02 |
| | 22 | 0.43 | 0.10 | 0.02 | 0.02 | 0.02 | 0.04 | 60.90 | 0.00 | 0.15 | 0.14 |
| | 30 | 0.37 | 0.12 | 0.03 | 0.05 | 0.06 | 0.05 | 74.03 | 0.00 | 0.17 | 0.19 |
| | 51 | 0.45 | 0.17 | 0.03 | 0.02 | 0.02 | 0.06 | 104.74 | 0.00 | 0.28 | 0.35 |
| | | | | | | | | | | | |
| **TrGA** | 5 | 0.03 | 0.00 | 0.02 | 0.06 | 0.01 | 0.00 | 9.13 | 0.00 | 0.04 | 0.00 |
| | 21 | 0.03 | 0.01 | 0.00 | 0.00 | 0.00 | 0.00 | 44.05 | 0.00 | 0.15 | 0.01 |
| | 29 | 0.04 | 0.01 | 0.00 | 0.00 | 0.00 | 0.00 | 55.46 | 0.00 | 0.17 | 0.01 |
| | 52 | 0.04 | 0.01 | 0.00 | 0.00 | 0.00 | 0.00 | 76.40 | 0.00 | 0.28 | 0.03 |
| | | | | | | | | | | | |
| **AnGA** | 5 | 0.03 | 0.00 | 0.01 | 0.04 | 0.01 | 0.01 | 9.82 | 0.00 | 0.04 | 0.00 |
| | 21 | 0.03 | 0.01 | 0.00 | 0.00 | 0.00 | 0.00 | 34.26 | 0.00 | 0.15 | 0.02 |
| | 29 | 0.03 | 0.01 | 0.00 | 0.00 | 0.00 | 0.00 | 34.96 | 0.00 | 0.17 | 0.03 |
| | 52 | 0.03 | 0.01 | 0.00 | 0.00 | 0.00 | 0.00 | 24.52 | 0.00 | 0.28 | 0.32 |

Data presented in Table 10 indicate HgGA combined with AmyE resulted in the highest lactic acid yield, while AnGA combined with AmyE resulted in the lowest lactic acid yield.

### Example 10: Comparison of various fermentation processes - glucose as substrate vs. conventional full saccharification process from starch

Glucose or the glucose syrup resulting from conventional liquefaction and full saccharification of cassava starch was used as the substrate for lactic acid fermentation. When glucose was used as the substrate, the inoculum of *Lactobacillus rhamnosus* was transferred to each 100 mL seed culture and cultivated at 37°C, 200rpm. Then 10 mL seed culture was added to each fermentor with 1 L fermentation medium. The fermentation temperature was controlled at 40°C. For the glucose syrup from conventional full saccharification of cassava starch, liquefaction was carried at 85°C for 90 min with 15% DS cassava starch, using alpha-amylase GC358 (Danisco US Inc., Genencor Division) at a dose of 0.25 kg/ton. When the mash was cool down to 60°C, saccharification was performed for about 18 h with 1 kg/ton AnGA. Fermentation was performed as using 15% glucose. Samples were withdrawn at various time points for HPLC analysis, and the results are presented in Table 11.

**Table 11. Comparison of lactic acid fermentation using (1) glucose and (2) the conventional full saccharification product of cassava starch**

| | **time(h)** | **DP >3 w/v%** | **DP-3 w**/**v**% | **DP-2 w**/**v**% | **Glucose w**/**v**% | **Fructose** %**w**/**v** | **succinic acid** %**w**/**v** | **lactic acid mg**/**mL** | **glycerol w**/**v**% | **acetic acid w/v%** | **ethanol w**/**v**% |
|---|---|---|---|---|---|---|---|---|---|---|---|
| **glucose** | 4 | 0.21 | 0.00 | 0.05 | 11.72 | 0.00 | 0.00 | 6.26 | 0.00 | 0.00 | 0.00 |
| | 21 | 0.28 | 0.11 | 0.07 | 1.85 | 0.00 | 0.04 | 74.73 | 0.00 | 0.07 | 0.19 |
| | 24 | 0.28 | 0.13 | 0.08 | 0.43 | 0.05 | 0.02 | 83.61 | 0.00 | 0.07 | 0.20 |
| | 28 | 0.28 | 0.12 | 0.07 | 0.01 | 0.05 | 0.03 | 81.92 | 0.00 | 0.08 | 0.25 |
| | 46 | 0.26 | 0.12 | 0.07 | 0.01 | 0.05 | 0.04 | 85.54 | 0.00 | 0.18 | 0.23 |
| | | | | | | | | | | | |
| **fully saccharified cassava starch** | 4 | 0.81 | 0.15 | 0.33 | 15.45 | 0.00 | 0.02 | 8.65 | 0.00 | 0.03 | 0.00 |
| | 21 | 0.92 | 0.26 | 0.31 | 5.14 | 0.00 | 0.03 | 82.61 | 0.00 | 0.03 | 0.05 |
| | 31 | 0.96 | 0.27 | 0.31 | 3.82 | 0.01 | 0.04 | 95.12 | 0.00 | 0.04 | 0.06 |
| | 45 | 0.91 | 0.26 | 0.29 | 1.10 | 0.07 | 0.04 | 111.70 | 0.00 | 0.05 | 0.07 |

Data presented in Table 8 indicate that there were still fermentable sugars remaining (1.10%) after 46 hours in the fermentation using conventional full saccharification product, while the glucose level dropped to 0.01% in the fermentation directly using glucose. Furthermore, compared with the fermentation directly using glucose, the fermentation using conventional full saccharification product resulted in a greater lactic acid yield and less amount of impurities (*i*.*e*., glycerol, acetic acid, and ethanol).

### Example 11. Comparison of various fermentation processes - SSF vs. glucose syrup from direct granular starch conversion

Three different processes using 15% DS cassava starch as the substrate for lactic acid fermentation were investigated. For SSF process, pH was adjusted to 6.5, liquefaction was carried at 85°C for 90 minutes using 15% DS cassava starch, and alpha-amylase GC358 (Danisco US Inc., Genencor Division) was dosed at 0.25 kg/t. 0.3 GAU/g TrGA was applied when the mash was cool down to 40°C for SSF. For the no-cook full saccharification process, pH was adjusted to 6.5, and 15% DS cassava starch was supplemented with 1.0 GAU/g HgGA and 0.5 kg/MT SPEZYME® XTRA (Danisco US Inc., Genencor Division). The saccharification was performed at 60°C for 18 hrs before fermentation. For the no-cook process, the slurry was adjusted to pH 6.5, supplemented with 2.0 GAU/g HgGA and 1.0 kg/MT AmyE, and then subject to fermentation directly. The inoculum of *Lactobacillus rhamnosus* was transferred to each 100 mL seed culture and cultivated at 37°C, 200 rpm. Then 10 mL seed culture was added to each fermentor with 1 L fermentation medium. The fermentation temperature was controlled at 40°C. Samples were withdrawn at various time points for HPLC analysis, and the results are presented in Table 12.

**Table 12. Comparison of lactic acid fermentation using (1) SSF, (2) no-cook full saccharification fermentation, and (2) no-cook direct fermentation**

| | **time(h)** | **DP >3 w/v%** | **DP-3 w/v%** | **DP-2 w/v%** | **Glucose w/v%** | **Fructose %w/v** | **succinic acid % w/v** | **lactic acid mg/mL** | **glycerol w/v%** | **acetic acid w/v%** | **ethanol w/v%** |
|---|---|---|---|---|---|---|---|---|---|---|---|
| **SSF** | 4 | 0.31 | 0.00 | 0.29 | 0.99 | 0.00 | 0.00 | 9.10 | 0.00 | 0.03 | 0.00 |
| | 21 | 0.15 | 0.03 | 0.04 | 0.32 | 0.00 | 0.00 | 88.73 | 0.00 | 0.04 | 0.04 |
| | 31 | 0.12 | 0.03 | 0.02 | 0.25 | 0.00 | 0.00 | 98.54 | 0.00 | 0.04 | 0.05 |
| | 45 | 0.06 | 0.02 | 0.01 | 0.07 | 0.01 | 0.00 | 116.97 | 0.00 | 0.03 | 0.06 |
| | | | | | | | | | | | |
| **No-cook full saccharification fermentation** | 0 | 1.41 | 0.00 | 1.16 | 11.21 | 0.00 | 0.00 | 0.00 | 0.00 | 0.02 | 0.00 |
| | 5 | 1.71 | 0.00 | 1.07 | 10.65 | 0.00 | 0.01 | 6.98 | 0.00 | 0.03 | 0.00 |
| | 21 | 1.20 | 0.38 | 0.52 | 0.09 | 0.09 | 0.05 | 89.66 | 0.00 | 0.00 | 0.04 |
| | 28 | 0.74 | 0.32 | 0.30 | 0.11 | 0.01 | 0.01 | 92.91 | 0.00 | 0.00 | 0.00 |
| | 52 | 0.96 | 0.38 | 0.13 | 0.01 | 0.00 | 0.03 | 96.28 | 0.00 | 0.00 | 0.05 |
| | | | | | | | | | | | |
| **No-cook direct fermentation** | 5 | 0.32 | 0.00 | 0.01 | 3.11 | 0.11 | 0.04 | 10.48 | 0.00 | 0.03 | 0.00 |
| | 22 | 0.44 | 0.11 | 0.03 | 0.03 | 0.05 | 0.06 | 74.87 | 0.00 | 0.08 | 0.13 |
| | 30 | 0.43 | 0.11 | 0.04 | 0.05 | 0.05 | 0.07 | 91.35 | 0.00 | 0.08 | 0.15 |
| | 51 | 0.47 | 0.12 | 0.02 | 0.00 | 0.02 | 0.05 | 116.37 | 0.00 | 0.11 | 0.30 |

Results presented in Table 9 indicate that the lactic acid yield was similar among all three processes. Traditionally liquefied cassava starch resulted in 116.97 mg/ml of lactic acid at 45 hours. For the cassava starch subject to the no-cook process, direct fermentation without saccharification resulted in 116.37 mg/ml of lactic acid at 51 hours, while fermentation of the fully saccharified substrate resulted in 96.28 mg/ml of lactic acid at 52 hours. The results further suggest that the no-cook process starting with raw starch may save at least 18 hours that was spent for saccharification.

### Example 12. Fermentation using starch substrates with a high dry solid (DS) value

The granular cornstarch slurry having a DS of 18%, 20%, or 25% was adjusted to pH 6.5, supplemented with 2.0 GAU/g HgGA and 1.0 kg/MT AmyE, and then subject to fermentation directly. For the fermentation, the inoculum of *Lactobacillus rhamnosus* was transferred to each 100 mL seed culture and cultivated at 37°C, 200 rpm. Then 10 mL seed culture was added to each fermentor with 1 L fermentation medium. The fermentation temperature was controlled at 40°C. Samples were withdrawn at various time points for HPLC analysis, and the results are presented in Table 13.

**Table 13. Lactic acid production using 18%, 20% and 25% DS cornstarch.**

| **DS** | **Time(h)** | **Glucose %w/v** | **lactic acid mg/mL** | **Productivity (g/L.h)** |
|---|---|---|---|---|
| **18%** | 5 | 4.74 | 8.75 | 1.75 |
| | 20 | 0.20 | 83.41 | 4.28 |
| | 28 | 0.13 | 103.62 | 3.70 |
| | 45 | 0.02 | 118.25 | 2.63 |
| | 53 | 0.02 | 122.89 | 2.34 |
| | 69 | 0.01 | 128.01 | 1.86 |
| | | | | |
| **20%** | 4 | 4.64 | 7.31 | 1.83 |
| | 22 | 0.13 | 89.27 | 4.15 |
| | 29 | 0.20 | 109.15 | 3.76 |
| | 45 | 0.05 | 125.88 | 2.80 |
| | 53 | 0.24 | 137.60 | 2.60 |
| | 117 | 0.05 | 144.36 | 1.23 |
| **25%** | 4 | 3.58 | 7.71 | 1.93 |
| | 22 | 0.24 | 102.40 | 4.76 |
| | 29 | 0.48 | 118.89 | 4.10 |
| | 45 | 1.16 | 137.15 | 3.05 |
| | 53 | 4.01 | 151.25 | 2.85 |
| | 117 | 0.53 | 164.94 | 1.41 |

The data presented in Table 13 indicate that HgGA combined with AmyE could be used to ferment lactic acid directly from granular starch having a high DS. This observation further suggests an increase of plant capacity without additional capital cost. As shown in Table 13, even at a high DS (25% ds), the rate of lactic acid production reached 4.76 g/L per hour, at 22 hrs.

### Example 13. Fermentation under various pH conditions

The no-cook direct fermentation process was further performed at pH 6.5 and pH 7.0. The slurry was adjusted to pH 6.5 or pH 7.0, supplemented with 1.5 GAU/g TrGA and 1.0 kg/MT SPEZYME® XTRA, and then subject to fermentation directly. The inoculum of *Lactobacillus rhamnosus* was transferred to each 100 mL seed culture and cultivated at 37°C, 200 rpm. Then 10 mL seed culture was added to each fermentor with 1 L fermentation medium. The fermentation temperature was controlled at 40°C. Samples were withdrawn at various time points for HPLC analysis, and the results are presented in Table 14.

**Table 14. Lactic acid production at pH 6.5 and pH 7.0.**

| | **time(h)** | **DP >3 w/v%** | **DP-3 w/v%** | **DP-2 w/v%** | **Glucose w/v%** | **Fructose %w/v** | **succinic acid % w/v** | **lactic acid mg/mL** | **acetic acid w/v%** | **ethanol w/v%** |
|---|---|---|---|---|---|---|---|---|---|---|
| **pH 6.5** | 4 | 0.73 | 0.00 | 0.17 | 4.20 | 0.03 | 0.03 | 7.98 | 0.02 | 0.00 |
| | 22 | 1.01 | 0.32 | 0.27 | 0.52 | 0.08 | 0.07 | 87.71 | 0.03 | 0.00 |
| | 29 | 0.74 | 0.31 | 0.13 | 0.83 | 0.08 | 0.08 | 97.57 | 0.03 | 0.00 |
| | 45 | 0.60 | 0.27 | 0.11 | 0.36 | 0.00 | 0.08 | 112.13 | 0.03 | 0.03 |
| | 53 | 0.52 | 0.25 | 0.10 | 0.28 | 0.00 | 0.09 | 117.45 | 0.03 | 0.03 |
| | 70 | 0.43 | 0.23 | 0.08 | 0.24 | 0.00 | 0.08 | 120.23 | 0.03 | 0.00 |
| | | | | | | | | | | |
| **pH 7.0** | 5 | 0.77 | 0.00 | 0.49 | 3.11 | 0.01 | 0.02 | 8.42 | 0.02 | 0.00 |
| | 22 | 0.81 | 0.29 | 0.06 | 0.34 | 0.03 | 0.06 | 91.60 | 0.04 | 0.04 |
| | 28 | 0.73 | 0.28 | 0.04 | 0.17 | 0.01 | 0.03 | 103.02 | 0.03 | 0.04 |
| | 45 | 0.50 | 0.24 | 0.04 | 0.24 | 0.02 | 0.06 | 118.11 | 0.02 | 0.04 |
| | 52 | 0.38 | 0.18 | 0.03 | 0.39 | 0.02 | 0.06 | 120.56 | 0.02 | 0.04 |
| | 69 | 0.36 | 0.19 | 0.03 | 0.24 | 0.02 | 0.06 | 124.86 | 0.04 | 0.04 |

As shown in Table 14, the lactic acid production results over time were similar at pH 6.5 and pH 7.0. The data suggest that the operation pH for the no-cook direct fermentation can be flexible, for example, in the range of 6.5 to 7.0.

### Example 14. No-cook process adapted to glucose-based strains

Two lactic acid-producing microorganism, *Bacillus coagulans* and *Lactobacillus rhamnosus,* were used ferment lactic acid with the no-cook process. The cornstarch slurry was adjusted to pH 6.5, supplemented with 1 GAU/g HgGA and 1.0 kg/MT AmyE, and then subject to fermentation directly. The inoculum of *Bacillus coagulans* or *Lactobacillus rhamnosus* was transferred to each 100 mL seed culture and cultivated at 37°C, 200 rpm. Then 10 mL seed culture was added to each fermentor with 1 L fermentation medium. The fermentation temperature was controlled at 40°C. Fermentation was also performed as using 15% glucose.
Samples were withdrawn at various time points for HPLC analysis, and the results are presented in Table 15.

**Table 15. Lactic acid production by different microorganism using different feed stocks**

| **Microorganism** | **Substrate Process** | **Time(h)** | **Glucose %w/v** | **lactic acid mg/mL** | **Productivity (g/L.h)** |
|---|---|---|---|---|---|
| ***Bacillus coagulans*** | **15% glucose convention process** | 5 | 13.89 | 7.38 | 1.48 |
| | | 22 | 11.22 | 19.19 | 0.87 |
| | | 29 | 6.72 | 48.47 | 1.67 |
| | **Cornstarch No-cook** | 5 | 3.33 | 8.29 | 1.66 |
| | | 22 | 1.90 | 38.80 | 1.76 |
| | | 29 | 1.08 | 53.93 | 1.86 |
| | | | | | |
| ***Lactobacillus rhamnosus*** | **15% glucose convention process** | 4 | 11.72 | 6.16 | 1.76 |
| | | 21 | 1.85 | 74.63 | 3.55 |
| | | 28 | 0.01 | 81.81 | 2.92 |
| | **Cornstarch** | 3 | 1.69 | 8.67 | 3.47 |
| | **No-cook** | 19 | 0.40 | 78.44 | 4.13 |
| | | 27 | 0.20 | 98.49 | 3.65 |

The data presented in Table 15 indicate that no-cook process resulted in (1) a higher lactic acid yield, and (2) a lower or at least equivalent level of residual glucose, for both *Bacillus coagulans* and *Lactobacillus rhamnosu.* This observation suggests that the no-cook process could adapt to glucose-based strains.

### Example 15: Succinic acid fermentation using HgGA

The following is a prophetic example. This experiment will be carried out in 1L bioreactor to monitor succinic acid formation from granular starch using enzymes with glucoamylase activity mentioned in Example 5, at desired fermentation conditions of pH 6.7 and temperature 34°C. For this experiment, raw granular starch in slurry form (maximum final concentration 80 g/L glucose) in 0.5 x TM2 fermentation medium, will be pasteurized (*i.e.* the mixture held at 34°C for 30 min for germination of any contaminant present in the starch slurry, and then pasteurized at 65°C for 14 hr). The pasteurized starch will be added to the pre-sterilized 1L bioreactor. The pH of the starch slurry plus medium will be adjusted to 6.7 and controlled at 6.65 with NH₄OH. Then, the desired enzymes mentioned in Example 6 will be added as 0.2 micron filtered solution (20ml) in DI water. An inoculum of succinic acid-producing strain 36 1.6ppc *E. coli,* taken from frozen vial, will be prepared in TM2 + 10g/L glucose medium. After the inoculum grows to OD 3-4, measured at 550 nm, 70 ml will be added to the bioreactor. At 3.7 hours into the run, the air being sparged at 0.6 slpm will be switched to nitrogen gas at 0.6 slpm. During the fermentation, samples will be taken from the vessel, centrifuged and the supernatants will be refrigerated to terminate the enzyme action. The supernatants will be subjected to HPLC analysis to estimate the bioconversion of granular starch by measuring glucose formation and its conversion to succinate at 34°C and pH 6.7.

### Example 16: 1,3-propanediol fermentation using HgGA

The following is a prophetic example. This experiment will be carried out in 1L fermentor to monitor 1,3-propanediol formation from granular starch using enzymes with glucoamylase activity at the desired fermentation pH 6.7 and temperature 34°C. For this experiment, granular starch in slurry form (for maximum final concentration 100 g/L glucose) in 0.5 x TM2 fermentation medium will be pasteurized (the slurry mixture held at 34°C for 30 min for germination of any contaminant present in the starch slurry, and then inactivated at 65°C for 14 hr). Then, pasteurized starch will be added to the pre-sterilized 1L fermentor. The pH of the slurry plus fermentation medium will be adjusted to 6.7 and controlled at 6.65 with NH₄OH. Then, the desired enzyme activity and requirements specific for 1,3-propanediol production (30 mg spectinomycin and 2 mg vitamin B12) will be added in DI water. An inoculum of 1,3-propanediol-producing *E. coli* strain TTaldABml/p109F1 taken from a frozen vial, will be prepared in soytone-yeast extract-glucose medium. After the inoculum grows to OD 3-4 (measured at 550 nm), 70 ml will be transferred to the 1L fermentor. During the fermentation, samples will be taken from the fermentor, centrifuged, and supernatants will be subjected to HPLC analysis. This will determine the fermentative bioconversion of granular starch by measuring glucose formation and its conversion to glycerol (1,3-propanediol pathway intermediate) and then to 1,3-propanediol.

### SEQUENCE LISTING

A Sequence Listing, comprising SEQ ID NOs: 1-9, is provided herein.
**SEQ ID NO: 1:** genomic sequence coding the full-length *Humicola grisea* glucoamylase; putative introns are in bold; corresponds to SEQ ID NO. 1 of U.S. Patent 7,262,041 
atgcatacct tctccaagct cctcgtcctg ggctctgccg tccagtctgc cctcgggcgg 60
cctcacggct cttcgcgtct ccaggaacgc gctgccgttg ataccttcat caacaccgag 120
aagcccatcg catggaacaa gctgctcgcc aacatcggcc ctaacggcaa agccgctccc 180
ggtgccgccg ccggcgttgt gattgccagc ccttccagga cggaccctcc ttgtacgtgg 240
tggcatggaa tggacccaag agactggttt tagatgaaag agagtttctg ctaaccgcca 300
cacccagact tcttcacctg gacccgcgat gccgccctgg tcctcaccgg catcatcgag 360
tcccttggcc acaactacaa caccaccctg cagaccgtca tccagaacta cgtcgcgtcg 420
caggccaagc tgcagcaggt ctcgaacccc tcgggaacct tcgccgacgg ctcgggtctc 480
ggtgaggcca agttcaatgt cgacctcact gccttcactg gcgaatgggg tcgccctcag 540
agggacggcc cgcccctgcg cgccatcgct ctcatccagt acgccaagtg gctgatcgcc 600
aacggctaca agagcacggc caagagcgtc gtctggcccg tcgtcaagaa cgatctcgcc 660
tacacggccc agtactggaa cgagaccggc ttcgatctct gggaggaggt ccccggcagc 720
tcgttcttta ccatcgccag ctctcacagg ggtgagtcat ttattgttca gtgttttctc 780
attgaataat taccggaatg ccactgacgc caaacagctc tgactgaggg tgcttacctc 840
gccgctcagc tcgacaccga gtgccgcgcc tgcacgaccg tcgcccctca ggttctgtgc 900
ttccagcagg ccttctggaa ctccaagggc aactatgtcg tctccaacag taagatccct 960
acaccaacaa aaaaaatcga aaaggaacgt tagctgaccc ttctagtcaa cggcggcgag 1020
tatcgctccg gcaaggacgc caactcgatc ctggcgtcca tccacaactt cgaccctgag 1080
gccggctgcg acaacctgac cttccagccc tgcagcgagc gcgccctggc caaccacaag 1140
gcctatgtcg actcgttccg caacctctac gccatcaaca agggcatcgc ccagggcaag 1200
gccgttgccg tcggccgcta ctcggaggat gtctactaca acggcaaccc gtggtacctg 1260
gccaactttg ccgccgccga gcagctctac gacgccatct acgtgtggaa caagcagggc 1320
tccatcaccg tgacctcggt ctccctgccc ttcttccgcg accttgtctc gtcggtcagc 1380
accggcacct actccaagag cagctcgacc ttcaccaaca tcgtcaacgc cgtcaaggcc 1440
tacgccgacg gcttcatcga ggtggcggcc aagtacaccc cgtccaacgg cgcgctcgcc 1500
gagcagtacg accgcaacac gggcaagccc gactcggccg ccgacctgac gtggtcgtac 1560
tcggccttcc tctcggccat cgaccgccgc gcgggtctcg tccccccgag ctggcgggcc 1620
agcgtggcca agagccagct gccgtccacc tgctcgcgca tcgaggtcgc cggcacctac 1680
gtcgccgcca cgagcacctc gttcccgtcc aagcagaccc cgaacccctc cgcggcgccc 1740
tccccgtccc cctacccgac cgcctgcgcg gacgctagcg aggtgtacgt caccttcaac 1800
gagcgcgtgt cgaccgcgtg gggcgagacc atcaaggtgg tgggcaacgt gccggcgctg 1860
gggaactggg acacgtccaa ggcggtgacc ctgtcggcca gcgggtacaa gtcgaatgat 1920
cccctctgga gcatcacggt gcccatcaag gcgacgggct cggccgtgca gtacaagtat 1980
atcaaggtcg gcaccaacgg gaagattact tgggagtcgg accccaacag gagcattacc 2040
ctgcagacgg cgtcgtctgc gggcaagtgc gccgcgcaga cggtgaatga ttcgtggcgt 2100
taa 2103
**SEQ ID NO: 2:** amino acid sequence of full-length *Humicola grisea* glucoamylase; the native signal sequence is in bold; corresponds to SEQ ID NO. 2 of U.S. Patent 7,262,041 
  1 MHTFSKLLVL GSAVQSALGR PHGSSRLQER AAVDTFINTE KPIAWNKLLA
NIGPNGKAAP
 61 GAAAGVVIAS PSRTDPPYFF TWTRDAALVL TGIIESLGHN YNTTLQTVIQ
NYVASQAKLQ
 
121 QVSNPSGTFA DGSGLGEAKF NVDLTAFTGE WGRPQRDGPP LRAIALIQYA
KWLIANGYKS
181 TAKSVVWPVV KNDLAYTAQY WNETGFDLWE EVPGSSFFTI ASSHRALTEG
AYLAAQLDTE
241 CRACTTVAPQ VLCFQQAFWN SKGNYVVSNI NGGEYRSGKD ANSILASIHN
FDPEAGCDNL
301 TFQPCSERAL ANHKAYVDSF RNLYAINKGI AQGKAVAVGR YSEDVYYNGN
PWYLANFAAA
361 EQLYDAIYVW NKQGSITVTS VSLPFFRDLV SSVSTGTYSK SSSTFTNIVN
AVKAYADGFI
421 EVAAKYTPSN GALAEQYDRN TGKPDSAADL TWSYSAFLSA IDRRAGLVPP
SWRASVAKSQ
481 LPSTCSRIEV AGTYVAATST SFPSKQTPNP SAAPSPSPYP TACADASEVY
VTFNERVSTA
541 WGETIKVVGN VPALGNWDTS KAVTLSASGY KSNDPLWSIT VPIKATGSAV
QYKYIKVGTN
601 GKITWESDPN RSITLQTASS AGKCAAQTVN DSWR
**SEQ ID NO: 3:** amino acid sequence of mature *Humicola grisea* glucoamylase; the native signal sequence is cleaved; corresponds to SEQ ID NO. 3 of U.S. Patent 7,262,041 
1 AAVDTFINTE KPIAWNKLLA NIGPNGKAAP GAAAGVVIAS PSRTDPPYFF
TWTRDAALVL
61 TGIIESLGHN YNTTLQTVIQ NYVASQAKLQ QVSNPSGTFA DGSGLGEAKF
NVDLTAFTGE
121 WGRPQRDGPP LRAIALIQYA KWLIANGYKS TAKSVVWPVV KNDLAYTAQY
WNETGFDLWE
181 EVPGSSFFTI ASSHRALTEG AYLAAQLDTE CRACTTVAPQ VLCFQQAFWN
SKGNYVVSNI
241 NGGEYRSGKD ANSILASIHN FDPEAGCDNL TFQPCSERAL ANHKAYVDSF
RNLYAINKGI
301 AQGKAVAVGR YSEDVYYNGN PWYLANFAAA EQLYDAIYVW NKQGSITVTS
VSLPFFRDLV
361 SSVSTGTYSK SSSTFTNIVN AVKAYADGFI EVAAKYTPSN GALAEQYDRN
TGKPDSAADL
421 TWSYSAFLSA IDRRAGLVPP SWRASVAKSQ LPSTCSRIEV AGTYVAATST
SFPSKQTPNP
481 SAAPSPSPYP TACADASEVY VTFNERVSTA WGETIKVVGN VPALGNWDTS
KAVTLSASGY
541 KSNDPLWSIT VPIKATGSAV QYKYIKVGTN GKITWESDPN RSITLQTASS
AGKCAAQTVN
601 DSWR
**SEQ ID NO: 4:** *Trichoderma reesei* glucoamylase cDNA
   <210> 4
   <211> 1899
   <212> DNA
   <213> Trichoderma reesei
   <400> 4 
atgcacgtcc tgtcgactgc ggtgctgctc ggctccgttg ccgttcaaaa ggtcctggga 60
agaccaggat caagcggtct gtccgacgtc accaagaggt ctgttgacga cttcatcagc 120
accgagacgc ctattgcact gaacaatctt ctttgcaatg ttggtcctga tggatgccgt 180
gcattcggca catcagctgg tgcggtgatt gcatctccca gcacaattga cccggactac 240
tattacatgt ggacgcgaga tagcgctctt gtcttcaaga acctcatcga ccgcttcacc 300
gaaacgtacg atgcgggcct gcagcgccgc atcgagcagt acattactgc ccaggtcact 360
ctccagggcc tctctaaccc ctcgggctcc ctcgcggacg gctctggtct cggcgagccc 420
aagtttgagt tgaccctgaa gcctttcacc ggcaactggg gtcgaccgca gcgggatggc 480
ccagctctgc gagccattgc cttgattgga tactcaaagt ggctcatcaa caacaactat 540
cagtcgactg tgtccaacgt catctggcct attgtgcgca acgacctcaa ctatgttgcc 600
cagtactgga accaaaccgg ctttgacctc tgggaagaag tcaatggagc ctcattcttt 660
actgttgcca accagcaccg agcacttgtc gagggcgcca ctcttgctgc cactcttggc 720
cagtcgggaa gcgcttattc atctgttgct ccccaggttt tgtgctttct ccaacgattc 780
tgggtgtcgt ctggtggata cgtcgactcc aacatcaaca ccaacgaggg caggactggc 840
aaggatgtca actccgtcct gacttccatc cacaccttcg atcccaacct tggctgtgac 900
gcaggcacct tccagccatg cagtgacaaa gcgctctcca acctcaaggt tgttgtcgac 960
tccttccgct ccatctacgg cgtgaacaag ggcattcctg ccggtgctgc cgtcgccatt 1020
ggccggtatg cagaggatgt gtactacaac ggcaaccctt ggtatcttgc tacatttgct 1080
gctgccgagc agctgtacga tgccatctac gtctggaaga agacgggctc catcacggtg 1140
accgccacct ccctggcctt cttccaggag cttgttcctg gcgtgacggc cgggacctac 1200
tccagcagct cttcgacctt taccaacatc atcaacgccg tctcgacata cgccgatggc 1260
ttcctcagcg aggctgccaa gtacgtcccc gccgacggtt cgctggccga gcagtttgac 1320
cgcaacagcg gcactccgct gtctgcgctt cacctgacgt ggtcgtacgc ctcgttcttg 1380
acagccacgg cccgtcgggc tggcatcgtg cccccctcgt gggccaacag cagcgctagc 1440
acgatcccct cgacgtgctc cggcgcgtcc gtggtcggat cctactcgcg tcccaccgcc 1500
acgtcattcc ctccgtcgca gacgcccaag cctggcgtgc cttccggtac tccctacacg 1560
cccctgccct gcgcgacccc aacctccgtg gccgtcacct tccacgagct cgtgtcgaca 1620
cagtttggcc agacggtcaa ggtggcgggc aacgccgcgg ccctgggcaa ctggagcacg 1680
agcgccgccg tggctctgga cgccgtcaac tatgccgata accaccccct gtggattggg 1740
acggtcaacc tcgaggctgg agacgtcgtg gagtacaagt acatcaatgt gggccaagat 1800
ggctccgtga cctgggagag tgatcccaac cacacttaca cggttcctgc ggtggcttgt 1860
gtgacgcagg ttgtcaagga ggacacctgg cagtcgtaa 1899
**SEQ ID NO: 5:** *Trichoderma reesei* glucoamylase, full length; with signal peptide
   <210> 1
   <211> 632
   <212> PRT
   <213> Trichoderma reesei
   <400> 1 
Met His Val Leu Ser Thr Ala Val Leu Leu Gly Ser Val Ala Val Gln
1               5                   10                  15
Lys Val Leu Gly Arg Pro Gly Ser Ser Gly Leu Ser Asp Val Thr Lys
            20                  25                  30
Arg Ser Val Asp Asp Phe Ile Ser Thr Glu Thr Pro Ile Ala Leu Asn
        35                  40                  45
Asn Leu Leu Cys Asn Val Gly Pro Asp Gly Cys Arg Ala Phe Gly Thr
    50                  55                  60
Ser Ala Gly Ala Val Ile Ala Ser Pro Ser Thr Ile Asp Pro Asp Tyr
65                  70                  75                  80
Tyr Tyr Met Trp Thr Arg Asp Ser Ala Leu Val Phe Lys Asn Leu Ile
                85                  90                  95
Asp Arg Phe Thr Glu Thr Tyr Asp Ala Gly Leu Gln Arg Arg Ile Glu
            100                 105                 110
Gln Tyr Ile Thr Ala Gln Val Thr Leu Gln Gly Leu Ser Asn Pro Ser
        115                 120                 125
Gly Ser Leu Ala Asp Gly Ser Gly Leu Gly Glu Pro Lys Phe Glu Leu
    130                 135                 140
Thr Leu Lys Pro Phe Thr Gly Asn Trp Gly Arg Pro Gln Arg Asp Gly
145                 150                 155                 160
Pro Ala Leu Arg Ala Ile Ala Leu Ile Gly Tyr Ser Lys Trp Leu Ile
                165                 170                 175
 
Asn Asn Asn Tyr Gln Ser Thr Val Ser Asn Val Ile Trp Pro Ile Val
            180                 185                 190
Arg Asn Asp Leu Asn Tyr Val Ala Gln Tyr Trp Asn Gln Thr Gly Phe
        195                 200                 205
Asp Leu Trp Glu Glu Val Asn Gly Ser Ser Phe Phe Thr Val Ala Asn
    210                 215                 220
Gln His Arg Ala Leu Val Glu Gly Ala Thr Leu Ala Ala Thr Leu Gly
225                 230                 235                 240
Gln Ser Gly Ser Ala Tyr Ser Ser Val Ala Pro Gln Val Leu Cys Phe
                245                 250                 255
Leu Gln Arg Phe Trp Val Ser Ser Gly Gly Tyr Val Asp Ser Asn Ile
            260                 265                 270
Asn Thr Asn Glu Gly Arg Thr Gly Lys Asp Val Asn Ser Val Leu Thr
        275                 280                 285
Ser Ile His Thr Phe Asp Pro Asn Leu Gly Cys Asp Ala Gly Thr Phe
    290                 295                 300
Gln Pro Cys Ser Asp Lys Ala Leu Ser Asn Leu Lys Val Val Val Asp
305                 310                 315                 320
Ser Phe Arg Ser Ile Tyr Gly Val Asn Lys Gly Ile Pro Ala Gly Ala
                325                 330                 335
Ala Val Ala Ile Gly Arg Tyr Ala Glu Asp Val Tyr Tyr Asn Gly Asn
            340                 345                 350
Pro Trp Tyr Leu Ala Thr Phe Ala Ala Ala Glu Gln Leu Tyr Asp Ala
        355                 360                 365
Ile Tyr Val Trp Lys Lys Thr Gly Ser Ile Thr Val Thr Ala Thr Ser
    370                 375                 380
Leu Ala Phe Phe Gln Glu Leu Val Pro Gly Val Thr Ala Gly Thr Tyr
385                 390                 395                 400
Ser Ser Ser Ser Ser Thr Phe Thr Asn Ile Ile Asn Ala Val Ser Thr
                405                 410                 415
Tyr Ala Asp Gly Phe Leu Ser Glu Ala Ala Lys Tyr Val Pro Ala Asp
            420                 425                 430
Gly Ser Leu Ala Glu Gln Phe Asp Arg Asn Ser Gly Thr Pro Leu Ser
        435                 440                 445
Ala Leu His Leu Thr Trp Ser Tyr Ala Ser Phe Leu Thr Ala Thr Ala
    450                 455                 460
Arg Arg Ala Gly Ile Val Pro Pro Ser Trp Ala Asn Ser Ser Ala Ser
465                 470                 475                 480
Thr Ile Pro Ser Thr Cys Ser Gly Ala Ser Val Val Gly Ser Tyr Ser
                485                 490                 495
Arg Pro Thr Ala Thr Ser Phe Pro Pro Ser Gln Thr Pro Lys Pro Gly
            500                 505                 510
Val Pro Ser Gly Thr Pro Tyr Thr Pro Leu Pro Cys Ala Thr Pro Thr
        515                 520                 525
Ser Val Ala Val Thr Phe His Glu Leu Val Ser Thr Gln Phe Gly Gln
    530                 535                 540
Thr Val Lys Val Ala Gly Asn Ala Ala Ala Leu Gly Asn Trp Ser Thr
545                 550                 555                 560
Ser Ala Ala Val Ala Leu Asp Ala Val Asn Tyr Ala Asp Asn His Pro
                565                 570                 575
Leu Trp Ile Gly Thr Val Asn Leu Glu Ala Gly Asp Val Val Glu Tyr
            580                 585                 590
Lys Tyr Ile Asn Val Gly Gln Asp Gly Ser Val Thr Trp Glu Ser Asp
        595                 600                 605
Pro Asn His Thr Tyr Thr Val Pro Ala Val Ala Cys Val Thr Gln Val
    610                 615                 620
Val Lys Glu Asp Thr Trp Gln Ser
625                 630
**SEQ ID NO: 6:** *Trichoderma reesei* glucoamylase, mature protein; without signal peptide 
<210>  2
<211>  599
<212>  PRT
<213>  Trichoderma reesei
 <400> 2 
Ser Val Asp Asp Phe Ile Ser Thr Glu Thr Pro Ile Ala Leu Asn Asn
1               5                   10                  15
Leu Leu Cys Asn Val Gly Pro Asp Gly Cys Arg Ala Phe Gly Thr Ser
            20                  25                  30
Ala Gly Ala Val Ile Ala Ser Pro Ser Thr Ile Asp Pro Asp Tyr Tyr
        35                  40                  45
Tyr Met Trp Thr Arg Asp Ser Ala Leu Val Phe Lys Asn Leu Ile Asp
    50                  55                  60
Arg Phe Thr Glu Thr Tyr Asp Ala Gly Leu Gln Arg Arg Ile Glu Gln
65                  70                  75                  80
Tyr Ile Thr Ala Gln Val Thr Leu Gln Gly Leu Ser Asn Pro Ser Gly
                85                  90                  95
Ser Leu Ala Asp Gly Ser Gly Leu Gly Glu Pro Lys Phe Glu Leu Thr
            100                 105                 110
Leu Lys Pro Phe Thr Gly Asn Trp Gly Arg Pro Gln Arg Asp Gly Pro
        115                 120                 125
Ala Leu Arg Ala Ile Ala Leu Ile Gly Tyr Ser Lys Trp Leu Ile Asn
    130                 135                 140
Asn Asn Tyr Gln Ser Thr Val Ser Asn Val Ile Trp Pro Ile Val Arg
145                 150                 155                 160
Asn Asp Leu Asn Tyr Val Ala Gln Tyr Trp Asn Gln Thr Gly Phe Asp
                165                 170                 175
Leu Trp Glu Glu Val Asn Gly Ser Ser Phe Phe Thr Val Ala Asn Gln
            180                 185                 190
His Arg Ala Leu Val Glu Gly Ala Thr Leu Ala Ala Thr Leu Gly Gln
        195                 200                 205
Ser Gly Ser Ala Tyr Ser Ser Val Ala Pro Gln Val Leu Cys Phe Leu
    210                 215                 220
Gln Arg Phe Trp Val Ser Ser Gly Gly Tyr Val Asp Ser Asn Ile Asn
225                 230                 235                 240
Thr Asn Glu Gly Arg Thr Gly Lys Asp Val Asn Ser Val Leu Thr Ser
                245                 250                 255
Ile His Thr Phe Asp Pro Asn Leu Gly Cys Asp Ala Gly Thr Phe Gln
            260                 265                 270
Pro Cys Ser Asp Lys Ala Leu Ser Asn Leu Lys Val Val Val Asp Ser
        275                 280                 285
Phe Arg Ser Ile Tyr Gly Val Asn Lys Gly Ile Pro Ala Gly Ala Ala
    290                 295                 300
Val Ala Ile Gly Arg Tyr Ala Glu Asp Val Tyr Tyr Asn Gly Asn Pro
305                 310                 315                 320
Trp Tyr Leu Ala Thr Phe Ala Ala Ala Glu Gln Leu Tyr Asp Ala Ile
                325                 330                 335
Tyr Val Trp Lys Lys Thr Gly Ser Ile Thr Val Thr Ala Thr Ser Leu
            340                 345                 350
Ala Phe Phe Gln Glu Leu Val Pro Gly Val Thr Ala Gly Thr Tyr Ser
        355                 360                 365
Ser Ser Ser Ser Thr Phe Thr Asn Ile Ile Asn Ala Val Ser Thr Tyr
    370                 375                 380
Ala Asp Gly Phe Leu Ser Glu Ala Ala Lys Tyr Val Pro Ala Asp Gly
385                 390                 395                 400
Ser Leu Ala Glu Gln Phe Asp Arg Asn Ser Gly Thr Pro Leu Ser Ala
                405                 410                 415
Leu His Leu Thr Trp Ser Tyr Ala Ser Phe Leu Thr Ala Thr Ala Arg
            420                 425                 430
Arg Ala Gly Ile Val Pro Pro Ser Trp Ala Asn Ser Ser Ala Ser Thr
        435                 440                 445
Ile Pro Ser Thr Cys Ser Gly Ala Ser Val Val Gly Ser Tyr Ser Arg
    450                 455                 460
Pro Thr Ala Thr Ser Phe Pro Pro Ser Gln Thr Pro Lys Pro Gly Val
465                 470                 475                 480
Pro Ser Gly Thr Pro Tyr Thr Pro Leu Pro Cys Ala Thr Pro Thr Ser
                485                 490                 495
Val Ala Val Thr Phe His Glu Leu Val Ser Thr Gln Phe Gly Gln Thr
            500                 505                 510
 
Val Lys Val Ala Gly Asn Ala Ala Ala Leu Gly Asn Trp Ser Thr Ser
        515                 520                 525
Ala Ala Val Ala Leu Asp Ala Val Asn Tyr Ala Asp Asn His Pro Leu
    530                 535                 540
Trp Ile Gly Thr Val Asn Leu Glu Ala Gly Asp Val Val Glu Tyr Lys
545                 550                 555                 560
Tyr Ile Asn Val Gly Gln Asp Gly Ser Val Thr Trp Glu Ser Asp Pro
                565                 570                 575
Asn His Thr Tyr Thr Val Pro Ala Val Ala Cys Val Thr Gln Val Val
            580                 585                 590
Lys Glu Asp Thr Trp Gln Ser
        595
**SEQ ID NO: 7:** *Trichoderma reesei* glucoamylase catalytic domain, 1-453 of mature TrGA, catalytic domain
   <210> 3
   <211> 453
   <212> PRT
   <213> Trichoderma reesei
   <400> 3 
Ser Val Asp Asp Phe Ile Ser Thr Glu Thr Pro Ile Ala Leu Asn Asn
1               5                   10                  15
Leu Leu Cys Asn Val Gly Pro Asp Gly Cys Arg Ala Phe Gly Thr Ser
            20                  25                  30
Ala Gly Ala Val Ile Ala Ser Pro Ser Thr Ile Asp Pro Asp Tyr Tyr
        35                  40                  45
Tyr Met Trp Thr Arg Asp Ser Ala Leu Val Phe Lys Asn Leu Ile Asp
    50                  55                  60
Arg Phe Thr Glu Thr Tyr Asp Ala Gly Leu Gln Arg Arg Ile Glu Gln
65                  70                  75                  80
Tyr Ile Thr Ala Gln Val Thr Leu Gln Gly Leu Ser Asn Pro Ser Gly
                85                  90                  95
Ser Leu Ala Asp Gly Ser Gly Leu Gly Glu Pro Lys Phe Glu Leu Thr
            100                 105                 110
Leu Lys Pro Phe Thr Gly Asn Trp Gly Arg Pro Gln Arg Asp Gly Pro
        115                 120                 125
Ala Leu Arg Ala Ile Ala Leu Ile Gly Tyr Ser Lys Trp Leu Ile Asn
    130                 135                 140
Asn Asn Tyr Gln Ser Thr Val Ser Asn Val Ile Trp Pro Ile Val Arg
145                 150                 155                 160
Asn Asp Leu Asn Tyr Val Ala Gln Tyr Trp Asn Gln Thr Gly Phe Asp
                165                 170                 175
Leu Trp Glu Glu Val Asn Gly Ser Ser Phe Phe Thr Val Ala Asn Gln
            180                 185                 190
His Arg Ala Leu Val Glu Gly Ala Thr Leu Ala Ala Thr Leu Gly Gln
        195                 200                 205
Ser Gly Ser Ala Tyr Ser Ser Val Ala Pro Gln Val Leu Cys Phe Leu
    210                 215                 220
Gln Arg Phe Trp Val Ser Ser Gly Gly Tyr Val Asp Ser Asn Ile Asn
225                 230                 235                 240
Thr Asn Glu Gly Arg Thr Gly Lys Asp Val Asn Ser Val Leu Thr Ser
                245                 250                 255
Ile His Thr Phe Asp Pro Asn Leu Gly Cys Asp Ala Gly Thr Phe Gln
            260                 265                 270
Pro Cys Ser Asp Lys Ala Leu Ser Asn Leu Lys Val Val Val Asp Ser
        275                 280                 285
Phe Arg Ser Ile Tyr Gly Val Asn Lys Gly Ile Pro Ala Gly Ala Ala
    290                 295                 300
Val Ala Ile Gly Arg Tyr Ala Glu Asp Val Tyr Tyr Asn Gly Asn Pro
305                 310                 315                 320
Trp Tyr Leu Ala Thr Phe Ala Ala Ala Glu Gln Leu Tyr Asp Ala Ile
                325                 330                 335
 
Tyr Val Trp Lys Lys Thr Gly Ser Ile Thr Val Thr Ala Thr Ser Leu
            340                 345                 350
Ala Phe Phe Gln Glu Leu Val Pro Gly Val Thr Ala Gly Thr Tyr Ser
        355                 360                 365
Ser Ser Ser Ser Thr Phe Thr Asn Ile Ile Asn Ala Val Ser Thr Tyr
    370                 375                 380
Ala Asp Gly Phe Leu Ser Glu Ala Ala Lys Tyr Val Pro Ala Asp Gly
385                 390                 395                 400
Ser Leu Ala Glu Gln Phe Asp Arg Asn Ser Gly Thr Pro Leu Ser Ala
                405                 410                 415
Leu His Leu Thr Trp Ser Tyr Ala Ser Phe Leu Thr Ala Thr Ala Arg
            420                 425                 430
Arg Ala Gly Ile Val Pro Pro Ser Trp Ala Asn Ser Ser Ala Ser Thr
        435                 440                 445
Ile Pro Ser Thr Cys
    450
**SEQ ID NO: 8:** native RhGA (P07683.2 GI:1168453) 
  1 mqlfnlplkv sfflvlsyfs llvsaasips sasvqldsyn ydgstfsgki yvkniayskk
 61 vtviyadgsd nwnnngntia asysapisgs nyeywtfsas ingikefyik yevsgktyyd
121 nnnsanyqvs tskpttttat attttapsts tttppsrsep atfptgnsti sswikkqegi
181 srfamlrnin ppgsatgfia aslstagpdy yyawtrdaal tsnvivyeyn ttlsgnktil
241 nvlkdyvtfs vktqststvc nclgepkfnp dasgytgawg rpqndgpaer attfilfads
301 yltqtkdasy vtgtlkpaif kdldyvvnvw sngcfdlwee vngvhfytlm vmrkglllga
361 dfakrngdst rastysstas tiankissfw vssnnwiqvs qsvtggvskk gldvstllaa
421 nlgsvddgff tpgsekilat avavedsfas lypinknlps ylgnsigryp edtyngngns
481 qgnswflavt gyaelyyrai kewignggvt vssislpffk kfdssatsgk kytvgtsdfn
541 nlaqnialaa drflstvqlh ahnngslaee fdrttglstg ardltwshas litasyakag
601 apaa
**SEQ ID NO: 9:** mature RhGA (P07683.2 GI:1168453)

1 asipssasvq ldsynydgst fsgkiyvkni ayskkvtviy adgsdnwnnn gntiaasysa
61 pisgsnyeyw tfsasingik efyikyevsg ktyydnnnsa nyqvstskpt tttatatttt
121 apststttpp srsepatfpt gnstisswik kqegisrfam lrninppgsa tgfiaaslst
181 agpdyyyawt rdaaltsnvi vyeynttlsg nktilnvlkd yvtfsvktqs tstvcnclge
241 pkfnpdasgy tgawgrpqnd gpaerattfi lfadsyltqt kdasyvtgtl kpaifkdldy
301 vvnvwsngcf dlweevngvh fytlmvmrkg lllgadfakr ngdstrasty sstastiank
361 issfwvssnn wiqvsqsvtg gvskkgldvs tllaanlgsv ddgfftpgse kilatavave
421 dsfaslypin knlpsylgns igrypedtyn gngnsqgnsw flavtgyael yyraikewig
481 nggvtvssis lpffkkfdss atsgkkytvg tsdfnnlaqn ialaadrfls tvqlhahnng
541 slaeefdrtt glstgardlt wshaslitas yakagapaa

## Claims

1. A method of processing starch in a
simultaneous saccharification and fermentation
(SSF) process comprising saccharifying a starch substrate to fermentable sugars at pH 6.0 to 7.5 in the presence of a glucoamylase, wherein the glucoamylase possesses at least 50% activity at pH 6.0 or above relative to its maximum activity, wherein the glucoamylase is *Humicola grisea* glucoamylase (HgGA) comprising SEQ ID NO: 3, or a variant thereof having at least 99% sequence identity to SEQ ID NO: 3, and fermenting the fermentable sugars to an end product.

2. The method of claim 1, wherein the variant has one amino acid modification compared to the parent glucoamylase.

3. The method of claim 1 or claim 2, wherein the HgGA is SEQ ID NO: 3.

4. The method of claim 1, wherein the SSF process is carried out at a pH between 7.0 to 7.5.

5. The method of any one of the preceding claims, wherein saccharifying is performed at a temperature in a range of 30°C to 60°C,

6. The method of claim 5, wherein saccharifying is performed at a temperature in a range of 40°C to 60°C.

7. The method of any one of the preceding claims, wherein the starch substrate is 15% to 50% dry solid (DS).

8. The method of claim 1, wherein the end product is selected from the group consisting of methanol, ethanol, butanol, monosodium glutamate, succinic acid, 1,3-propanediol, vitamins, amino acids, and lactic acid.

9. The method of claim 8, wherein the end product is ethanol, 1,3-propanediol, or succinic acid.

10. The method of any one of the preceding claims, wherein the starch substrate is granular starch or liquefied starch.

11. The method of any one of the preceding claims, wherein the glucoamylase is dosed at a range of 0.1 to 2.0 GAU per gram of dry substance starch.

12. The method of any one of the preceding claims further comprising adding an alpha-amylase.

13. The method of claim 12 , wherein the alpha-amylase is from a *Bacillus* species,

14. The method of claim 13, wherein the alpha-amylase is from a *Bacillus subtilis* alpha-amylase (AmyE), a *Bacillus amyloliquefaciens* alpha-amylase, a *Bacillus licheniformis* alpha-amylase or a *Bacillus stearothermophilus* alpha-amylase.

15. The method of any one of the preceding claims, wherein the starch substrate is from corn, wheat, rye, barley, sorghum, cassava, tapioca, potato and any combination thereof.

16. The method of any one of the preceding claims which further comprises at least one other enzyme selected from the group consisting of proteases, pullulanases, isoamylases, cellulases, hemicellulases, xylanases, cyclodextrin glycotransferases, lipases, phytases, laccases, oxidases, esterases, cutinases, xylanases, and alpha-glucosidases.

17. The method of any one of the preceding claims which further comprises at least one other non-starch polysaccharide hydrolyzing enzyme selected from the group consisting of cellulases, hemicellulases and pectinases.

## Patentansprüche

1. Verfahren zum Verarbeiten von Stärke in einem Prozess zur gleichzeitigen Verzuckerung und Fermentation (SSF), umfassend Verzuckern eines Stärkesubstrats zu fermentierbaren Zuckern bei pH 6,0 bis 7,5 in Gegenwart einer Glucoamylase, wobei die Glucoamylase mindestens 50% Aktivität bei pH 6,0 oder darüber relativ zu ihrer maximalen Aktivität besitzt, wobei die Glucoamylase *Humicola-grisea-*Glucoamylase (HgGA), umfassend SEQ ID NO: 3, oder eine Variante davon mit mindestens 99% Sequenzidentität zu SEQ ID NO: 3, ist, und Fermentieren der fermentierbaren Zucker zu einem Endprodukt.

2. Verfahren nach Anspruch 1, wobei die Variante eine Aminosäuremodifizierung, verglichen mit der Eltern-Glucoamylase, hat.

3. Verfahren nach Anspruch 1 oder Anspruch 2, wobei die HgGA SEQ ID NO: 3 ist.

4. Verfahren nach Anspruch 1, wobei der SSF-Prozess bei einem pH zwischen 7,0 bis 7,5 ausgeführt wird.

5. Verfahren nach einem der vorhergehenden Ansprüche, wobei das Verzuckern bei einer Temperatur in einem Bereich von 30°C bis 60°C durchgeführt wird.

6. Verfahren nach Anspruch 5, wobei das Verzuckern bei einer Temperatur in einem Bereich von 40°C bis 60°C durchgeführt wird.

7. Verfahren nach einem der vorhergehenden Ansprüche, wobei das Stärkesubstrat zu 15% bis 50% trockener Feststoff (DS) ist.

8. Verfahren nach Anspruch 1, wobei das Endprodukt aus der Gruppe, bestehend aus Methanol, Ethanol, Butanol, Mononatriumglutamat, Succinsäure, 1,3-Propandiol, Vitaminen, Aminosäuren und Milchsäure, ausgewählt ist.

9. Verfahren nach Anspruch 8, wobei das Endprodukt Ethanol, 1,3-Propandiol oder Succinsäure ist.

10. Verfahren nach einem der vorhergehenden Ansprüche, wobei das Stärkesubstrat körnige Stärke oder verflüssigte Stärke ist.

11. Verfahren nach einem der vorhergehenden Ansprüche, wobei die Glucoamylase mit einem Bereich von 0,1 bis 2,0 GAU pro Gramm von trockener Stärkesubstanz dosiert wird.

12. Verfahren nach einem der vorhergehenden Ansprüche, weiterhin umfassend Hinzufügen von einer alpha-Amylase.

13. Verfahren nach Anspruch 12 , wobei die alpha-Amylase von einer *Bacillus-*Spezies ist.

14. Verfahren nach Anspruch 13, wobei die alpha-Amylase von einer *Bacillus*subtilis-alpha-Amylase (AmyE), einer *Bacillus-amyloliquefaciens-*alpha-Amylase*,* einer *Bacillus-licheniformis-alpha-Amylase* oder einer *Bacillus-stearothermophilus-*alpha-Amylase ist.

15. Verfahren nach einem der vorhergehenden Ansprüche, wobei das Stärkesubstrat aus Mais, Weizen, Roggen, Gerste, Sorghum, Maniok, Tapioka, Kartoffel und einer Kombination davon ist.

16. Verfahren nach einem der vorhergehenden Ansprüche, welches weiterhin mindestens ein anderes Enzym, ausgewählt aus der Gruppe, bestehend aus Proteasen, Pullulanasen, Isoamylasen, Cellulasen, Hemicellulasen, Xylanasen, Cyclodextrin-Glycotransferasen, Lipasen, Phytasen, Laccasen, Oxidasen, Esterasen, Cutinasen, Xylanasen und alpha-Glucosidasen, umfasst.

17. Verfahren nach einem der vorhergehenden Ansprüche, welches weiterhin mindestens ein anderes nicht-Stärke-Polysaccharid hydrolysierendes Enzym, ausgewählt aus der Gruppe, bestehend aus Cellulasen, Hemicellulasen und Pectinasen, umfasst.

## Revendications

1. Méthode pour la transformation d'amidon dans un procédé de saccharification et de fermentation simultanées (SSF) comprenant la saccharification d'un substrat d'amidon en sucres fermentables à pH 6,0 à 7,5 en présence d'une glucoamylase, dans laquelle la glucoamylase possède au moins 50% d'activité à pH 6,0 ou au-dessus relativement à son activité maximum, dans laquelle la glucoamylase est une glucoamylase de *Humicola grisea* (HgGA) comprenant la SEQ ID NO: 3 ou un variant de celle-ci possédant au moins 99% d'identité de séquence avec la SEQ ID NO: 3, et la fermentation des sucres fermentables en un produit final.

2. Méthode selon la revendication 1, dans laquelle le variant possède une modification d'acide aminé comparé à la glucoamylase parente.

3. Méthode selon la revendication 1 ou la revendication 2, dans laquelle la HgGA est la SEQ ID NO: 3.

4. Méthode selon la revendication 1, dans laquelle le procédé SSF est réalisé à un pH entre 7,0 et 7,5.

5. Méthode selon l'une quelconque des revendications précédentes, dans laquelle la saccharification est effectuée à une température dans un intervalle de 30°C à 60°C.

6. Méthode selon la revendication 5, dans laquelle la saccharification est effectuée à une température dans un intervalle de 40°C à 60°C.

7. Méthode selon l'une quelconque des revendications précédentes, dans laquelle le substrat d'amidon est de 15% à 50% de solide sec (DS).

8. Méthode selon la revendication 1, dans laquelle le produit final est choisi dans le groupe constitué de: méthanol, éthanol, butanol, glutamate de monosodium, acide succinique, 1,3-propanediol, vitamines, acides aminés et acide lactique.

9. Méthode selon la revendication 8, dans laquelle le produit final est l'éthanol, le 1,3-propanediol ou l'acide succinique.

10. Méthode selon l'une quelconque des revendications précédentes, dans laquelle le substrat d'amidon est un amidon granulaire ou un amidon liquéfié.

11. Méthode selon l'une quelconque des revendications précédentes, dans laquelle la glucoamylase est dosée à un intervalle de 0,1 à 2,0 GAU par gramme de substance sèche d'amidon.

12. Méthode selon l'une quelconque des revendications précédentes, comprenant en outre l'ajout d'une alpha-amylase.

13. Méthode selon la revendication 12, dans laquelle l'alpha-amylase est issue d'une espèce *Bacillus.*

14. Méthode selon la revendication 13, dans laquelle l'alpha-amylase est issue d'une alpha-amylase de *Bacillus subtilis* (AmyE), d'une alpha-amylase de *Bacillus amyloliquefaciens,* d'une alpha-amylase de *Bacillus licheniformis* ou d'une alpha-amylase de *Bacillus stearothermophilus.*

15. Méthode selon l'une quelconque des revendications précédentes, dans laquelle le substrat d'amidon est issu de maïs, blé, seigle, orge, sorgho, manioc, tapioca, pomme de terre et toute combinaison de ceux-ci.

16. Méthode selon l'une quelconque des revendications précédentes, qui comprend en outre au moins une autre enzyme choisie dans le groupe constitué de protéases, pullulanases, isoamylases, cellulases, hémicellulases, xylanases, cyclodextrine glycotransférases, lipases, phytases, laccases, oxydases, estérases, cutinases, xylanases et alpha-glucosidases.

17. Méthode selon l'une quelconque des revendications précédentes, qui comprend en outre au moins une autre enzyme hydrolysant un polysaccharide sans amidon choisie dans le groupe constitué de cellulases, hémicellulases et pectinases.
